# EUROPEAN PATENT APPLICATION

(11) **EP 1 746 096 A1**
(43) Date of publication of application: **24.01.2007**
(21) Application number: 05015432.7
(22) Date of filing: 15.07.2005
(51) Int. Cl.: C07D 417/12, C07D 417/14, C07D 473/34, C07D 487/04, C07D 495/04, A61K 31/428, A61P 35/00

(54) **2-Arylbenzothiazole analogues and uses thereof in the treatment of cancer**

(71) Applicant: 4SC AG, 82152 Martinsried (DE)
(72) Inventor: Ehlert, Jan, Dr., 79238 Ehrenkirchen (DE); Herz, Thomas, Dr., 82131 Stockdorf (DE); Krauss, Rolf, Dr., 82152 Planegg-Martinsried (DE); Kubbutat, Michael, Dr., 79227 Schallstadt (DE); Lang, Martin, Dr., 82166 Gräfelfing (DE); Saeb, Wael, Dr., 82152 Planegg-Martinsried (DE); Schächtele, Christoph, Dr., 79108 Freiburg (DE); Tasler, Stefan, Dr., 82205 Gilching (DE); Totzke, Frank, Dr., 79098 Freiburg (DE); Zirrgiebel, Ute, Dr., 79194 Gundelfingen (DE)
(74) Representative: Strehl Schübel-Hopf & Partner

(57) **Abstract**

The present invention relates to anticancer compounds of the general formula (I) and salts and physiologically functional derivatives thereof, 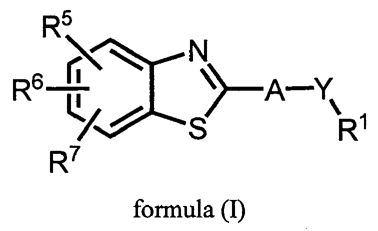 wherein
Y independently represents S, O, NR², SO, SO₂;
A independently represents a five- or six-membered aromatic carbocycle or heterocycle
and wherein R¹ in formula (I) represents one of the heteroaryl groups defined in the claims.

## Description

The present invention relates to 2-arylbenzothiazoles of the general formula (I) or a salt or a physiologically functional derivative or a stereoisomer thereof, for use as a medicament. The compounds of the invention are exceptionally useful for the treatment of diseases associated with abnormal and hyperproliferation of cells in a mammal, especially humans. In particular, they are useful for the treatment of all forms of cancer.
Furthermore a process of preparing said 2-arylbenzothiazole derivatives is disclosed.

### Background of the invention

Protein kinases play a central role in the regulation of cellular functions. These include processes like cell growth and division, cell differentiation and cell death, but also many other cellular activities. Protein kinases catalyze the transfer of phosphate residues from ATP on target proteins which as a consequence of this protein kinase mediated phosphorylation change their three-dimensional structure and thereby their physiological function. Depending on the amino acid which is phosphorylated by a protein kinase these enzymes are grouped in two families, the so-called serine/threonine protein kinases and the tyrosine protein kinases.
Based on the human genome project it is known that in human beings there exist 518 DNA sequences which encode for a protein kinase-like protein sequence. For several of these 518 proteins it could be shown in the last about 20 years that modifications in their related gene sequences (e.g. point mutations, deletions or gene amplifications) result in pathological changes of the cellular activities of the corresponding protein kinase. This is in particular true for protein kinases which are involved in cell proliferation and cell cycle control, in survival of cells and cell death, in tumor angiogenesis, and in formation of tumor metastases.
Several so-called oncogenes are pathologically modified genes which in their proto-oncogenic form encode for protein kinases involved in normal, physiological regulation of cell growth and division.
Since protein kinases are key regulators of cell functions and since they can show dysregulated enzymatic activity in cells they are promising targets for the development of therapeutic agents. There are many ongoing drug discovery projects in the pharmaceutical industry with the goal to identify modulators of protein kinases. The major focus is currently on protein kinases involved in inflammation and cancer, but besides this protein kinases are currently discussed as promising targets in almost every area of diseases. In the field of tumors the first protein kinase inhibitors (Gleevec, Iressa) have already reached the market. In addition, a great number of protein kinase inhibitors are currently in various phases of clinical development. In most cases these compounds are either targeting subtypes of the EGF (Epidermal Growth Factor) receptor family or of the VEGF (Vascular Endothelial Growth Factor) receptor family. All these compounds have been developed with the goal to specifically inhibit one particular protein kinase, for which there is evidence that it interferes with one of the four major molecular processes of tumor progression. These four processes are (1) cell proliferation/cell cycle control, (2) regulation of programmed cell death (apoptosis) and cell survival, (3) tumor angiogenesis and (4) tumor metastasis.
The present invention relates to 2-arylbenzothiazole derivatives which may be useful for inhibition of protein kinases involved in diseases besides cancer, but which are especially useful as anti-tumor agents. This includes monospecific protein kinase inhibitors, which preferentially inhibit one protein kinase which is causally involved in tumor progression, but also so-called multi-target protein kinase inhibitors, which inhibit at least two different protein kinases which either relate to the same or to two or more different molecular mechanisms of tumor progression. As an example, such a compound could be an inhibitor of tumor angiogenesis and, in addition, also a stimulator of apoptosis.
The concept of multi-target protein kinase inhibitors is a new approach although the idea of developing "multiplex protein kinase inhibitors" has already been described by J. Adams et al., Current Opinion in Chemical Biology 6, 486-492, 2002. Therein compounds are described, which, at the same time, inhibit several protein kinases, which however all are involved in one molecular mechanism of tumor progression, namely tumor angiogenesis.

The object of the present invention is solved by the subject-matter of the independent claims. Further advantageous features, aspects and details of the invention are evident from the dependent claims, the description, the figures, and the examples of the present application.
Considering the lack of currently available treatment options for the majority of the conditions associated with protein kinases like ABL1, AKT1, AKT2, AKT3, ARK5, Aurora-A, Aurora-B, Aurora-C, BRK, CDK1, CDK2, CDK3, CDK4, CDK5, CDK6, CDK7, CDK9, CHK1, CK2, COT, CSK, DAPK1, EGF-R, EPHA1, EPHA2, EPHA4, EPHB1, EPHB2, EPHB3, EPHB4, ERBB2, ERBB4, FAK, FGF-R1, FGF-R3, FGF-R4, FGR, FLT3, GSK3-beta, IGF1-R, IKK-beta, IKK-epsilon, INS-R, IRAK4, ITK, JAK2, JAK3, JNK3, KIT, LCK, LYN, MET, MST4, MUSK, NEK2, NEK6, NLK, PAK1, PAK2, PAK4, PBK, PCTAIRE1, PDGFR-alpha, PDGFR-beta, PDK1, PIM1, PIM2, PKC-alpha, PKC-beta1, PKC-beta2, PKC-delta, PKC-epsilon, PKC-eta, PKC-gamma, PKC-iota, PKC-mu, PKC-theta, PKC-zeta, PLK1, PRK1, RET, ROCK2, S6K, SAK, SGK1, SGK3, SNK, SRC, SYK, TIE2, TSF1, TSK2, VEGF-R1, VEGF-R2, VEGF-R3, VRK1, WEE1, YES, ZAP70 especially with protein kinases like EGF-R (cell proliferation), ERBB2 (cell proliferation), PDGFR (cell proliferation), Aurora-A (cell cycle control), Aurora-B (cell cycle control), IGF1-R (apoptosis), VEGF-R2 (angiogenesis), VEGF-R3 (angiogenesis), TIE2 (angiogenesis), EPHB4 (angiogenesis), and SRC kinase (metastasis), there is still a great need for new therapeutic agents that inhibit these protein targets.
2-Arylbenzothiazole derivatives described herein are a new group of protein kinase inhibitors which show differential inhibition of protein kinases, each of which can be assigned to one of the four molecular mechanisms of tumor development.

In W00149686 2-Phenyl-benzothiazoles substituted by an aminotriazine of the following general formula are described as UV-filters used as skin and hair sunscreens. 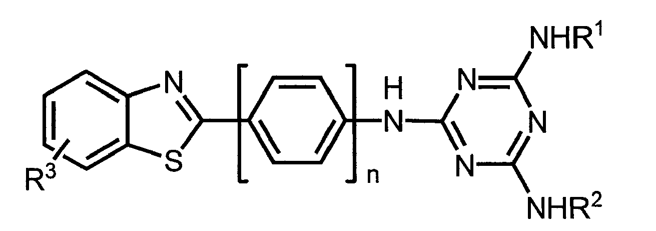 Herein n can be 0 or 1.

Similar compounds are described in W09825922, in EP841341 and in JP11060573, all of them also substituted by a aminotriazine.

In EP 711818 2-Phenyl-benzothiazoles are claimed as liquid crystal compositions.

The present invention relates to compounds of the general formula (I) or a salt or a physiologically functional derivative or a stereoisomer thereof, 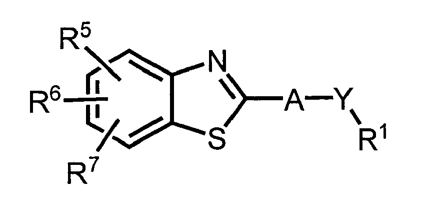 formula (I)
wherein
- Y: independently represents a divalent linkage selected from S, O, NR², SO, SO₂;
- A: independently represents a divalent linkage selected from a five- or six-membered aromatic carbocycle or heterocycle each of which is optionally substituted by one to four R³ or R⁴ and is, if Y is NR², together with Y not 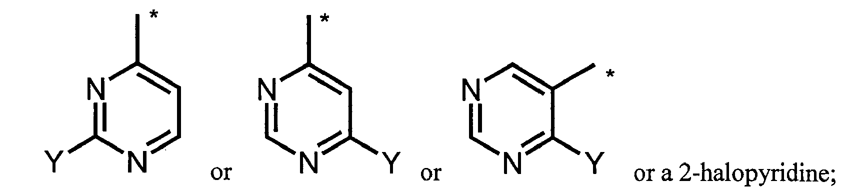
where * indicates the point of attachment to the benzothiazole;
- R¹: independently represents one of the following groups:

| | | |
|---|---|---|
| 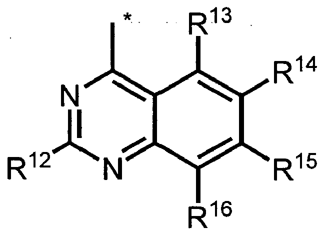 | 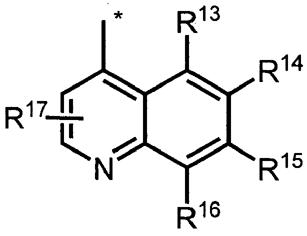 | 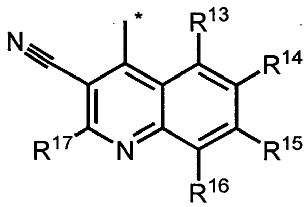 |
| 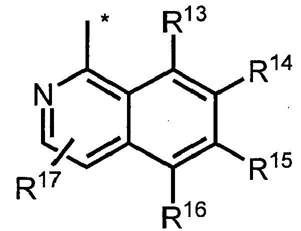 | 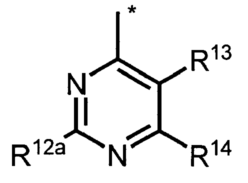 | 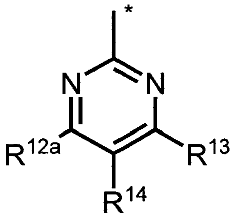 |
| 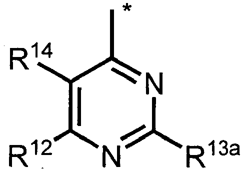 | 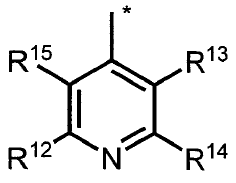 | 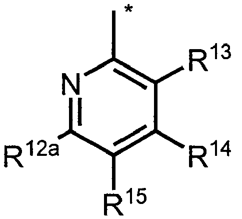 |
| 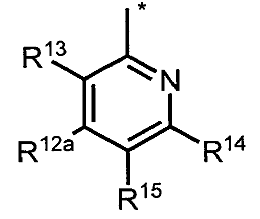 | 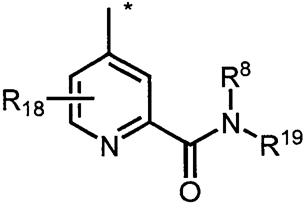 | 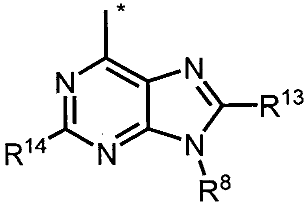 |
| 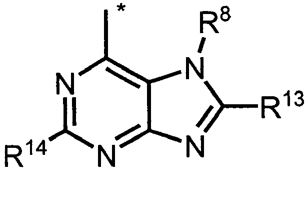 | 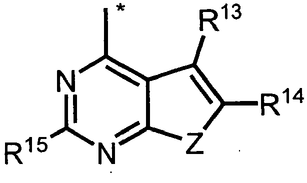 | 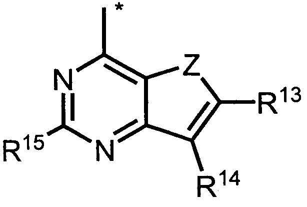 |
| 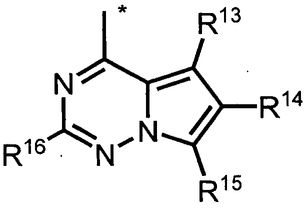 | | |

| | | |
|---|---|---|
| where * indicates the point of attachment | | |

Z independently represents O, NR⁸, S;
R² independently represents H, alkyl, cycloalkyl, -COR¹¹, -SOR¹¹, -SO₂R¹¹, -CN, hydroxyalkyl, haloalkyl, haloalkyloxy, oxyalkyl, alkoxyalkyl, aminoalkyl, alkylaminoalkyl, alkylamine, -Y^{2'}-X^{2'};
Y^{2'} independently represents C₁₋₆alkyl, C₁₋₃alkylcycloalkyl, alkyloxyalkyl, alkylaminoalkyl or alkylaryl;
X^{2'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
R³ independently represents H, -COR¹¹, CO₂R¹¹, -SOR¹¹, -SO₂R¹¹, -SO₃R¹¹, -NO₂,-CN, -CF₃, -OCH₃, -OCF₃, alkyl, cycloalkyl, alkoxy, oxyalkyl, alkoxyalkyl, -NH₂, alkylamine, aminoalkyl, alkylaminoalkyl, -NR⁸COR¹¹, halogen, -OH, -SH, alkylthio, hydroxyalkyl, haloalkyl, haloalkyloxy, or -Y^{3'}-X^{3'};
Y^{3'} independently represents C₁₋₆alkyl, C₁₋₃alkylcycloalkyl, alkyloxyalkyl, alkylaminoalkyl or alkylaryl;
X^{3'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
R⁴ independently represents H, -COR¹¹, -CO₂R¹¹, -SOR¹¹, -SO₂R¹¹, -SO₃R¹¹, -NO₂,-CN, -CF₃, -OCH₃, -OCF₃, alkyl, cycloalkyl, alkoxy, oxyalkyl, alkoxyalkyl, -NH₂, alkylamine, aminoalkyl, alkylaminoalkyl, -NR⁸COR¹¹, halogen, -OH, -SH, alkylthio, hydroxyalkyl, haloalkyl, haloalkyloxy, -Y^{4'}-X^{4'}, substituted or unsubstituted aryl or heteroaryl;
Y^{4'} independently represents C₁₋₆alkyl, C₁₋₃alkylcycloalkyl, alkyloxyalkyl, alkylaminoalkyl or alkylaryl;
X^{4'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
R⁵ independently represents H, -COR¹¹, -CO₂R¹¹, -SOR¹¹, -SO₂R¹¹, -SO₃R¹¹, -NO₂,-CN, -CF₃, -OCH₃, -OCF₃, alkyl, cycloalkyl, alkoxy, oxyalkyl, alkoxyalkyl, -NH₂, alkylamine, aminoalkyl, alkylaminoalkyl, -NR⁸COR¹¹, halogen, -OH, -SH, alkylthio, hydroxyalkyl, haloalkyl, haloalkyloxy, -Y^{5'}-X^{5'}, substituted or unsubstituted aryl or heteroaryl;
Y^{5'} independently represents C₁₋₆alkyl, C₁₋₃alkylcycloalkyl, alkyloxyalkyl, alkylaminoalkyl or alkylaryl;
X^{5'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
R⁶ independently represents H, -COR¹¹, -CO₂R¹¹, -SOR¹¹, -SO₂R¹¹, -SO₃R¹¹, -NO₂,-CN, -CF₃, -OCH₃, -OCF₃, alkyl, cycloalkyl, alkoxy, oxyalkyl, alkoxyalkyl, -NH₂, alkylamine, aminoalkyl, alkylaminoalkyl, -NR⁸COR¹¹, halogen, -OH, -SH, alkylthio, hydroxyalkyl, haloalkyl, haloalkyloxy, -Y^{6'}-X^{6'}, substituted or unsubstituted aryl or heteroaryl;
Y^{6'} independently represents C₁₋₆alkyl, C₁₋₃alkylcycloalkyl, alkyloxyalkyl, alkylaminoalkyl or alkylaryl;
X^{6'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl,
R⁷ -SO₂N(C₁₋₃alkyl)₂ -CN, -NO₂, -NH₂, -NHC₁₋₃alkyl, -N(C₁₋₃alkyl)₂, -OH, -SH; independently represents H, -COR¹¹, -CO₂R¹¹, -SOR¹¹, -SO₂R¹¹, -SO₃R¹¹, -NO₂,-CN, -CF₃, -OCH₃, -OCF₃, alkyl, cycloalkyl, alkoxy, oxyalkyl, alkoxyalkyl, -NH₂, alkylamine, aminoalkyl, alkylaminoalkyl, -NR⁸COR¹¹, halogen, -OH, -SH, alkylthio, hydroxyalkyl, haloalkyl, haloalkyloxy, -Y^{7'}-X^{7'}, substituted or unsubstituted aryl or heteroaryl;
Y^{7'} independently represents C₁₋₆alkyl, C₁₋₃alkylcycloalkyl, alkyloxyalkyl, alkylaminoalkyl or alkylaryl;
X^{7'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
R⁸ independently represents H, alkyl, cycloalkyl, -COR¹¹, -SOR¹¹, -SO₂R¹¹, hydroxyalkyl, haloalkyl, haloalkyloxy, oxyalkyl, alkoxyalkyl, aminoalkyl, alkylaminoalkyl, -Y^{8'}-X^{8'}, substituted or unsubstituted aryl or heteroaryl;
Y^{8'} independently represents C₁₋₆alkyl, C₁₋₃alkylcycloalkyl, alkyloxyalkyl, alkylaminoalkyl or alkylaryl;
X^{8'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
R⁹ independently represents H, alkyl, cycloalkyl, hydroxyalkyl, haloalkyl, haloalkyloxy, oxyalkyl, alkoxyalkyl, aminoalkyl, alkylaminoalkyl, aryl or heteroaryl, which may be substituted;
R¹¹ independently represents H, alkyl, cycloalkyl, -NR⁸R⁹, -NR⁸NR⁸R⁹, -ONR⁸R⁹, -NR⁸OR⁹, alkylamine, arylamine, -Y^{11'}-X^{11'}, substituted or unsubstituted aryl or heteroaryl;
Y^{11'} independently represents C₁₋₆alkyl, C₁₋₃alkylcycloalkyl, allcyloxyalkyl, alkylaminoalkyl or alkylaryl;
X^{11'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
R¹² independently represents H, -NHR⁸; or one of the groups: 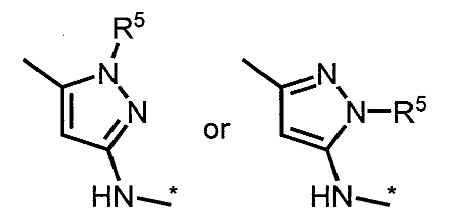 where * indicates the point of attachment.
R^{12a} independently represents one of the groups: 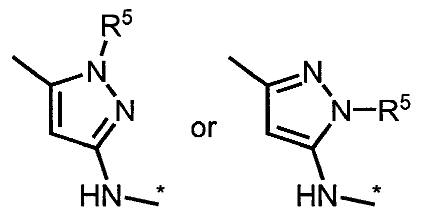 where * indicates the point of attachment.
R¹³ independently represents H, halogen, nitro, -CN, trifluoromethyl, alkyl, alkylaryl, arylalkyl, aryl, heteroaryl, -NR⁸R⁹, -Y^{13'}-X^{13'}, or -X²R¹⁸;
Y^{13'} independently represents C₁₋₆alkyl, C₁₋₃alkylcycloalkyl, alkyloxyalkyl, alkylaminoalkyl or alkylaryl;
X^{13'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂, -CN, -NO₂, -NH₂, -NHC₁₋₃alkyl, -N(C₁₋₃alkyl)₂, -OH, -SH;
R^{13a} independently represents H, halogen, nitro, -CN, trifluoromethyl, alkyl, alkylaryl, arylalkyl, aryl, heteroaryl, -Y^{13'}-X¹³;
R¹⁴ independently represents H, halogen, nitro, -CN, trifluoromethyl, alkyl, alkylaryl, arylalkyl, aryl, heteroaryl, -NR⁸R⁹, -Y^{14'}-X^{14'}, or -X²R¹⁸;
Y^{14'} independently represents C₁₋₆alkyl, C₁₋₃alkylcycloalkyl, alkyloxyalkyl, alkylaminoalkyl or alkylaryl;
X^{14'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂, -CN, -NO₂, -NH₂, -NHC₁₋₃alkyl, -N(C₁₋₃alkyl)₂, -OH, -SH;
R¹⁵ independently represents H, halogen, nitro, -CN, trifluoromethyl, alkyl, alkylaryl, arylalkyl, aryl, heteroaryl, -NR⁸R⁹, -Y^{15'}-X^{15'}, or -X²R¹⁸;
Y^{15'} independently represents C₁₋₆alkyl, C₁₋₃alkylcycloalkyl, alkyloxyalkyl, alkylaminoalkyl or alkylaryl;
X^{15'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂, -CN, -NO₂, -NH₂, -NHC₁₋₃alkyl, -N(C₁₋₃alkyl)₂, -OH, -SH;
R¹⁶ independently represents H, halogen, nitro, -CN, trifluoromethyl, alkyl, alkylaryl, arylalkyl, aryl, heteroaryl, -NR⁸R⁹, -Y^{16'}-X^{16'}, or -X²R¹⁸;
Y^{16'} independently represents C₁₋₆alkyl, C₁₋₃alkylcycloalkyl, alkyloxyalkyl, alkylaminoalkyl or alkylaryl;
X^{16'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂, -CN, -NO₂, -NH₂, -NHC₁₋₃alkyl, -N(C₁₋₃alkyl)₂, -OH, -SH;
R¹⁷ independently represents H, halogen, nitro, trifluoromethyl, alkyl, alkylaryl, arylalkyl, aryl, heteroaryl, -NR⁸R⁹ -Y^{17'}-X^{17'}, or -X²R¹⁸;
Y^{17'} independently represents C₁₋₆alkyl, C₁₋₃alkylcycloalkyl, alkyloxyalkyl, alkylaminoalkyl or alkylaryl;
X^{17'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
X² independently represents a direct bond, -O-, -CH₂-, -OCO-, carbonyl, -S-, -SO-, -SO₂-, -NR⁸CO-, -CONR⁸-, -SO₂NR⁸-, -NR⁸SO₂- or -NR⁸-;
R¹⁸ independently represents H, alkyl, cycloalkyl, -COR¹¹, -SOR¹¹, -SO₂R¹¹, -OCH₃,-OCF₃, hydroxyalkyl, haloalkyl, haloalkyloxy, oxyalkyl, alkoxyalkyl, aminoalkyl, alkylaminoalkyl, -Y^{18'}-X^{18'}, or one of the following groups:

| | | |
|---|---|---|
| 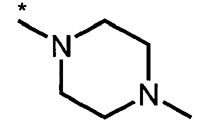 | 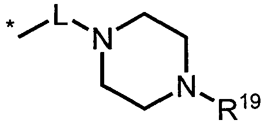 | 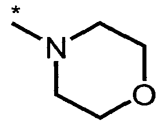 |
| 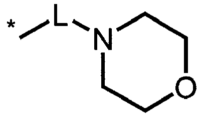 | 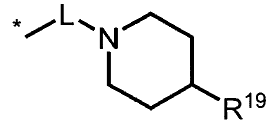 | 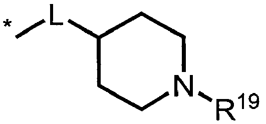 |
| 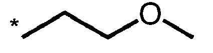 | 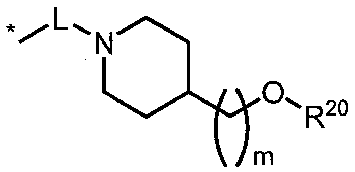 | 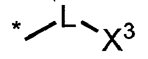 |
| 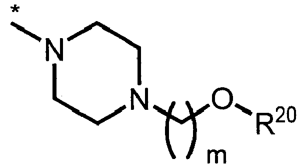 | 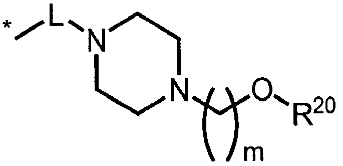 | 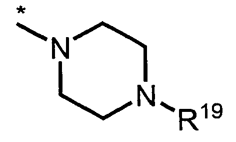 |
| 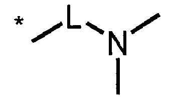 | 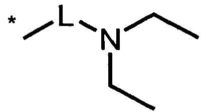 | 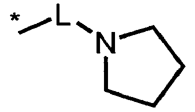 |
| 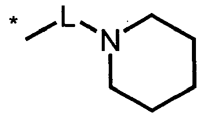 | 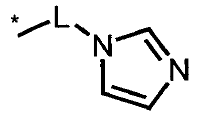 | 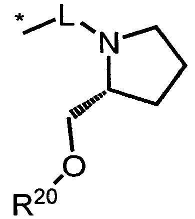 |
| 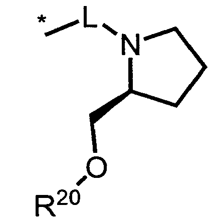 | 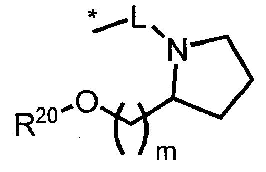 | 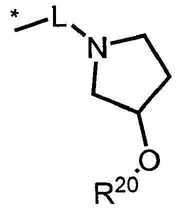 |
| 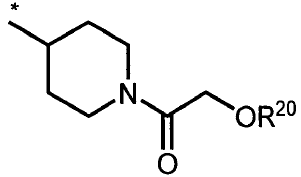 | 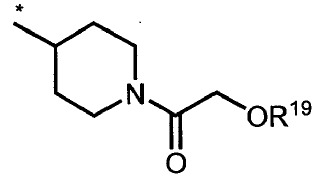 | |

| | | |
|---|---|---|
| where * indicates the point of attachment | | |

Y^{18'} independently represents C₁₋₆alkyl, C₁₋₃alkylcycloalkyl, alkyloxyalkyl, alkylaminoalkyl or alkylaryl;
X^{18'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
m independently represents an integer from 1-3;
L independently represents a direct bond, alkyl, cycloalkyl, alkylamine, alkyloxy, oxyalkyl, aminoalkyl, cycloalkyloxy, cycloalkylamine, ethyl, propyl, aryl or heteroaryl each of which unsubstituted or substituted;
X³ independently represents -COOH, -COOC₁₋₆alkyl, -CONR⁸R⁹, -OH, -NR⁸R⁹, -SH, -SO₃H, -SO₂NR⁸R⁹;
R¹⁹ independently represents H, alkyl, cycloalkyl, alkylamine, alkyloxy, oxyalkyl, aminoalkyl, cycloalkyloxy, cycloalkylamine;
R²⁰ H, -PO₃H₂; an alkyl group, if not stated otherwise, denotes a linear or branched C₁-C₆-alkyl, preferably a linear or branched chain of one to five carbon atoms, a linear or branched C₂-C₆-alkenyl or a linear or branched C₂-C₆-alkynyl group, which can be substituted by one or more substituents R';
the C₁-C₆-alkyl, C₂-C₆-alkenyl and C₂-C₆-alkynyl residue may be selected from the group comprising -CH₃, -C₂H₅, -CH=CH₂, -C=CH, -C₃H₇, -CH(CH₃)₂, -CH₂-CH=CH₂, -C(CH₃)=CH₂, -CH=CH-CH₃, -C≡C-CH₃, -CH₂-C≡CH, -C₄H₉, -CH₂-CH(CH₃)₂, -CH(CH₃)-C₂H₅, -C(CH₃)₃, -C₅H₁₁, -C₆H₁₃, -C(R')₃, -C₂(R')₅, -CH₂-C(R')₃, -C₃(R')₇, -C₂H₄-C(R')₃, -C₂H₄-CH=CH₂, -CH=CH-C₂H₅, -CH=C(CH₃)₂, -CH₂-CH=CH-CH₃, -CH=CH-CH=CH₂, -C₂H₄-C≡CH, -C≡C-C₂H₅, -CH₂-C≡C-CH₃, -C≡C-CH=CH₂, -CH=CH-C=CH, -C≡C-C≡CH, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂Hs, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, C(CH₃)=C(CH₃)₂, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C=CH, -CH₂-C≡C-C≡CH, -C≡C-CH=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C=CH, -CH=C(CH₃)-C≡CH, -C≡C-C(CH₃)=CH₂, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -C₄H₈-C≡CH, -C≡C-C₄H₉, -C₃H₆-C≡C-CH₃, -CH₂-C≡C-C₃H₇, -C₂H₄-C≡C-C₂H₅;

R' independently represents H, -CO₂R", -CONHR", -CR"O, -SO₂NR", -NR"-CO-haloalkyl, -NO₂, -NR"-SO₂-haloalkyl, -NR"-SO₂-alkyl, -SO₂-alkyl, -NR"-CO-alkyl, -CN, alkyl, cycloalkyl, aminoalkyl, alkylamino, alkoxy, -OH, -SH, alkylthio, hydroxyalkyl, hydroxyalkylamino, halogen, haloalkyl, haloalkyloxy, aryl, arylalkyl or heteroaryl;

R" independently represents H, haloalkyl, hydroxyalkyl, alkyl, cycloalkyl, aryl, heteroaryl or aminoalkyl;
a cycloalkyl group denotes a non-aromatic ring system containing three to eight carbon atoms, preferably four to eight carbon atoms, wherein one or more of the carbon atoms in the ring can be substituted by a group E, E being O, S, SO, SO₂, N, or NR", R" being as defined above; the C₃-C₈-cycloalkyl residue may be selected from the group comprising -cyclo-C₃H₅, -cyclo-C₄H₇, -cyclo-C₅H₉, -cyclo-C₆H₁₁, -cyclo-C₇H₁₃, -cyclo-C₈H₁₅, morpholine-4-yl, piperazinyl, 1-alkylpiperazine-4-yl;
an alkoxy group denotes an O-alkyl group, the alkyl group being as defined above; the alkoxy group is preferably a methoxy, ethoxy, isopropoxy, *t*-butoxy or pentoxy group;
an alkylthio group denotes an S-alkyl group, the alkyl group being as defined above;
an haloalkyl group denotes an alkyl group which is substituted by one to five halogen atoms, the alkyl group being as defined above; the haloalkyl group is preferably a -C(R¹⁰)₃, -CR¹⁰(R^{10'})₂, -CR¹⁰(R¹⁰)R^{10"}, -C₂(R¹⁰)₅, -CH₂-C(R¹⁰)₃, -CH₂-CR¹⁰(R¹⁰)₂, -CH₂-CR¹⁰(R^{10'})R^{10"}, -C₃(R¹⁰)₇, or -C₂H₄-C(R¹⁰)₃, wherein R¹⁰, R^{10'}, R^{10"} represent F, Cl, Br or I, preferably F;
a hydroxyalkyl group denotes an HO-alkyl group, the alkyl group being as defined above;
an haloalkyloxy group denotes an alkoxy group which is substituted by one to five halogen atoms, the alkyl group being as defined above; the haloalkyloxy group is preferably a -OC(R¹⁰)₃, -OCR¹⁰(R^{10'})₂, -OCR¹⁰(R^{10'})R^{10"}, -OC₂(R¹⁰)₅, -OCH₂-C(R¹⁰)₃, -OCH₂-CR¹⁰(R^{10'})₂, -OCH₂-CR¹⁰(R^{10'})R^{10"}, -OC₃(R¹⁰)₇ or -OC₂H₄-C(R¹⁰)₃, wherein R¹⁰, R^{10'}, R^{10"} represent F, Cl, Br or I, preferably F;
a hydroxyalkylamino group denotes an (HO-alkyl)₂-N- group or HO-alkyl-NH- group, the alkyl group being as defined above;
an alkylamino group denotes an HN-alkyl or N-dialkyl group, the alkyl group being as defined above;
a halogen group is fluorine, chlorine, bromine, or iodine;
an aryl group denotes an aromatic group having five to fifteen carbon atoms, which can be substituted by one or more substituents R', where R' is as defined above; the aryl group is preferably a phenyl group, -o-C₆H₄- R', -m-C₆H₄- R', -p-C₆H₄- R', 1-naphthyl, 2-naphthyl, 1-anthracenyl or 2-anthracenyl;
a heteroaryl group denotes a 5- or 6-membered heterocyclic group which contains at least one heteroatom like O, N, S. This heterocyclic group can be fused to another ring. For example, this group can be selected from a thiazol-2-yl, thiazol-4-yl, thiazol-5-yl, isothiazol-3-yl, isothiazol-4-yl, isothiazol-5-yl, 1,2,4-oxadiazol-3-yl, 1,2,4-oxadiazol-5-yl, 1,2,4-thiadiazol-3-yl, 1,2,4-thiadiazol-5-yl, 1,2,5-oxadiazol-3-yl, 1,2,5-oxadiazol-4-yl, 1,2,5-thiadiazol-3-yl, 1-imidazolyl, 2-imidazolyl, 1,2,5-thiadiazol-4-yl, 4-imidazolyl, 1-pyrrolyl, 2-pyrrolyl, 3-pyrrolyl, 2-furanyl, 3-furanyl, 2-thienyl, 3-thienyl, 2-pyridyl, 3-pyridyl, 4-pyridyl, 2-pyranyl, 3-pyranyl, 4-pyranyl, 2-pyrimidinyl, 4-pyrimidinyl, 5-pyrimidinyl, 3-pyridazinyl, 4-pyridazinyl, 2-pyrazinyl, 1-pyrazolyl, 3-pyrazolyl, 4-pyrazolyl, 1*H*-tetrazol-2-yl, 1*H*-tetrazol-3-yl, tetrazolyl, 2-indolyl, 3-indolyl, 2-indolinyl, 3-indolinyl, benzo[b]furanyl, benzo[b]thiophenyl, benzimidazolyl, benzothiazolyl, quinazolinyl, quinoxazolinyl, quinolinyl, tetrahydroquinolinyl, isoquinolinyl, or tetrahydroisoquinolinyl group. This heterocyclic group can be substituted by one or more substituents R', wherein R' is as defined above.

The invention also provides a pharmaceutical composition comprising a compound of formula (I), in free form or in the form of pharmaceutically acceptable salts and physiologically functional derivatives, together with a pharmaceutically acceptable diluent or carrier therefore.

The term "physiologically functional derivative" as used herein refers to compounds which are not pharmaceutically active themselves but which are transformed into their pharmaceutical active form in vivo, i.e. in the subject to which the compound is administered. Examples of physiologically functional derivatives are prodrugs such as those described below in the present application.

In addition, the present invention provides methods for preparing the compounds of the invention such as compounds of formula (I).

The compounds of formula (I) may be obtained *via* various methods. One possibility for the synthesis of compounds of formula (I) comprises the step of reacting a compound of formula (VII), wherein R⁵-R⁷, A, and Y are defined as above, with a compound of formula (VIII), wherein R¹ is as defined above and LG comprises a leaving group such as Cl, Br, and I. Either nucleophilic substitution or palladium-catalyzed cross-coupling may be applied. If Y = NR², R² may be added before or after addition of R¹.

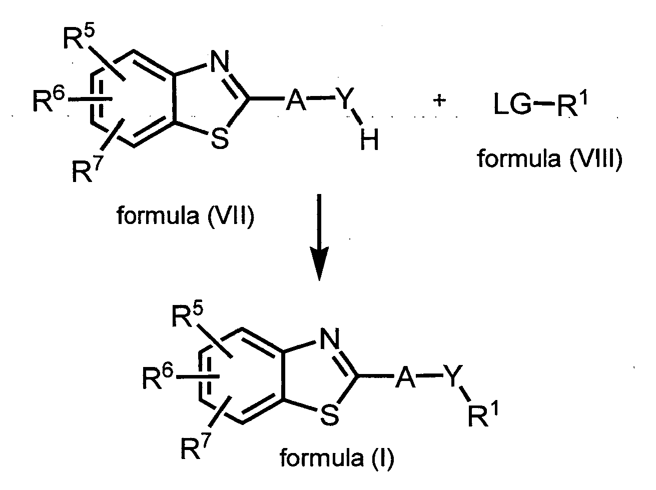

Compounds of formula (VII) can be synthesized by reaction of a thioamine according to formula (X) with an carboxylic acid of the formula (IX).

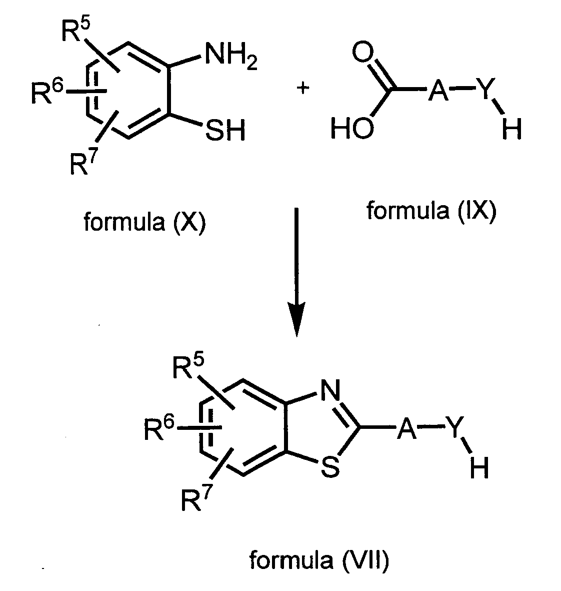

Alternatively an activated acid derivative (e.g. acid chloride) may be used instead of the acid according to formula (IX).

Another way to synthesize compounds of the formula (VII) is the conversion of compounds of formula (XI), a disulfide, into the benzothiazole of formula (VII) by reduction of the disulfide and subsequent condensation.

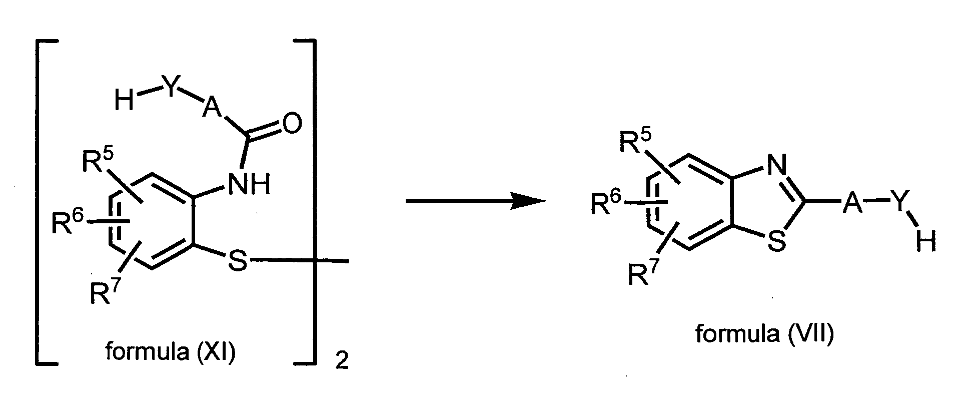

In some cases in the synthesis of compounds of the formula (VII) and formula (XI) Y may be protected. This protection group has to be removed before converting them into compounds of the formula (I). Carboxylic acids of formula (IX) may arise from nitrile hydrolysis.

Compounds of the formula (XI) may be obtained by reaction of a disulfide according to formula (XII) with a compound of formula (IX) or alternatively an activated carboxylic acid derivative.

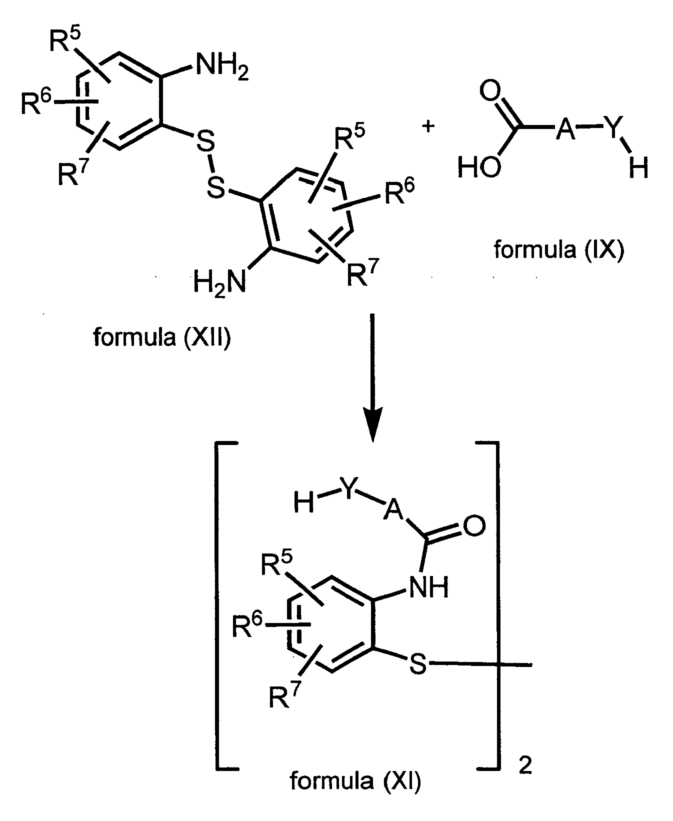

As far as compounds of the general formula (X) and formula (XII) could not be purchased, they were synthesized from the benzothiazoles, 2-aminobenzthiazoles or 2-methylbenzothiazoles of formula (XIII) e.g. by hydrolysis under basic conditions.

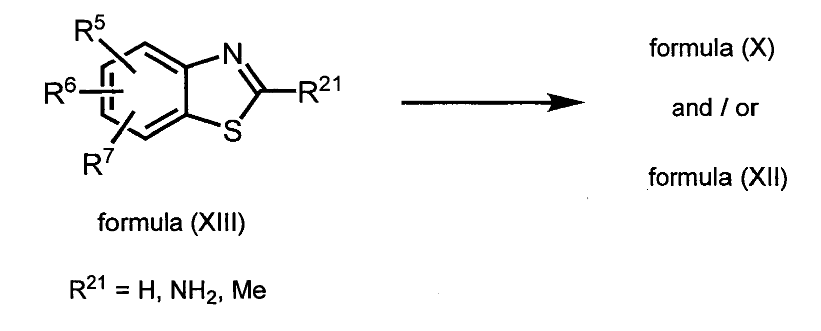

A preferred embodiment of the invention, in the compounds of formula (I), are compounds of the formula (II) 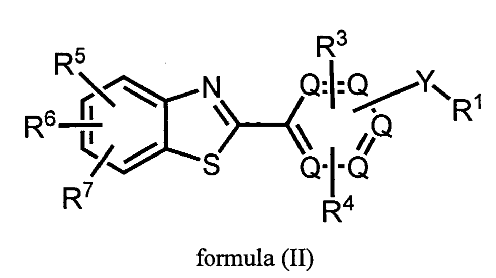 wherein
- Q: independently represents C, N, CH;
- Y: is as defined above;
- R¹⁻²⁰: are as defined above.

In a more preferred embodiment of the invention, in the compounds of formula (II) are the compounds where Q independently represents C or CH.

In an even more preferred embodiment of the invention, in the compounds of formula (I), are compounds of the formula (III) 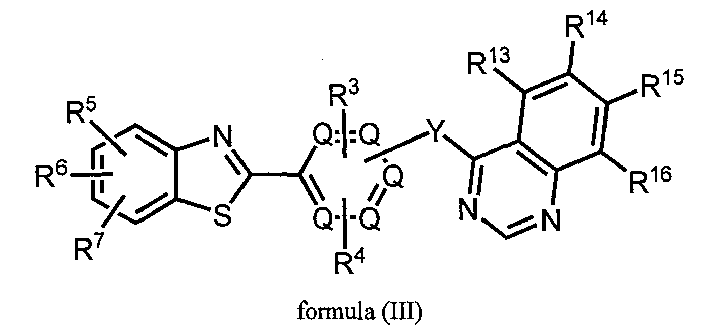 wherein
- Q: independently represents C, N, CH;
- Y: is as defined above;
- R²⁻²⁰: are as defined above.

Another preferred embodiment of the invention, in the compounds of formula (I), are compounds of the formula (IV) 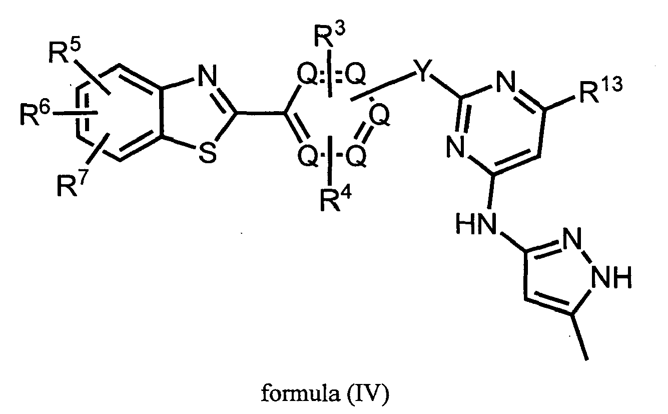 wherein
- Y: is as defined above;
- R²⁻²⁰: are as defined above.

Exemplary compounds of formula (I) of the present invention include, but are not limited to, the following:

| **Name** | **Compound** |
|---|---|
| (4-Benzothiazol-2-yl-phenyl)-(4,6-dimethyl-pyrimidin-2-yl)-amine | 1 |
| (4-Benzothiazol-2-yl-2-methyl-phenyl)-(4,6-dimethyl-pyrimidin-2-yl)-amine | 2 |
| (3-Benzothiazol-2-yl-phenyl)-(4,6-dimethyl-pyrimidin-2-yl)-amine | 3 |
| (5-Benzothiazol-2-yl-2-chloro-phenyl)-(4,6-dimethyl-pyrimidin-2-yl)-amine | 4 |
| (4-Benzothiazol-2-yl-3-methoxy-phenyl)-(4,6-dimethyl-pyrimidin-2-yl)-amine | 5 |
| 2-Benzothiazol-2-yl-5-(4,6-dimethyl-pyrimidin-2-ylamino)-phenol | 6 |
| 2-Benzothiazol-2-yl-4-(4,6-dimethyl-pyrimidin-2-ylamino)-phenol | 7 |
| (5-Benzothiazol-2-yl-2-methyl-phenyl)-(4,6-dimethyl-pyrimidin-2-yl)-amine | 8 |
| (4-Benzothiazol-2-yl-phenyl)-(6,7-dimethoxy-quinazolin-4-yl)-amine | 9 |
| (4-Benzothiazol-2-yl-phenyl)-[4-(4-methyl-piperazin-1-yl)-pyrimidin-2-yl]-amine | 10 |
| N2-(4-Benzothiazol-2-yl-phenyl)-N4-(5-methyl-1H-pyrazol-3-yl)-pyrimidine-2,4-diamine | 11 |
| (3-Benzothiazol-2-yl-phenyl)-(6,7-dimethoxy-quinazolin-4-yl)-amine | 12 |
| 2-Benzothiazol-2-yl-5-(6,7-dimethoxy-quinazolin-4-ylamino)-phenol | 13 |
| (4-Benzothiazol-2-yl-3-methoxy-phenyl)-(6,7-dimethoxy-quinazolin-4-yl)-amine | 14 |
| (4-Benzothiazol-2-yl-phenyl)-(9H-purin-6-yl)-amine | 15 |
| (3-Benzothiazol-2-yl-phenyl)-(9H-purin-6-yl)-amine | 16 |
| (4-Benzothiazol-2-yl-2-methyl-phenyl)-(6,7-dimethoxy-quinazolin-4-yl)-amine | 17 |
| (5-Benzothiazol-2-yl-2-chloro-phenyl)-(6,7-dimethoxy-quinazolin-4-yl)-amine | 18 |
| (5-Benzothiazol-2-yl-2-methyl-phenyl)-(6,7-dimethoxy-quinazolin-4-yl)-amine | 19 |
| 2-Benzothiazol-2-yl-4-(6,7-dimethoxy-quinazolin-4-ylamino)-phenol | 20 |
| (3-Benzothiazol-2-yl-phenyl)-[4-(4-methyl-piperazin-1-yl)-pyrimidin-2-yl]-amine | 21 |
| N2-(3-Benzothiazol-2-yl-phenyl)-N4-(5-methyl-1H-pyrazol-3-yl)-pyrimidine-2,4-diamine | 22 |
| N2-(4-Benzothiazol-2-yl-phenyl)-N4-methyl-pyrimidine-2,4-diamine | 23 |
| N4-(4-Benzothiazol-2-yl-phenyl)-N2-methyl-pyrimidine-2,4-diamine | 24 |
| N4-(4-Benzothiazol-2-yl-phenyl)-6,7-dimethoxy-N2-methyl-quinazoline-2,4-diamine | 25 |
| N4-(4-Benzothiazol-2-yl-phenyl)-6,7-dimethoxy-N2-(5-methyl-1H-pyrazol-3-yl)-quinazoline-2,4-diamine | 26 |
| N4-(3-Benzothiazol-2-yl-phenyl)-6,7-dimethoxy-N2-methyl-quinazoline-2,4-diamine | 27 |
| N2-(3-Benzothiazol-2-yl-phenyl)-N4-methyl-pyrimidine-2,4-diamine | 28 |
| N4-(3-Benzothiazol-2-yl-phenyl)-N2-methyl-pyrimidine-2,4-diamine | 29 |
| N4-(3-Benzothiazol-2-yl-phenyl)-6,7-dimethoxy-N2-(5-methyl-1H-pyrazol-3-yl)-quinazoline-2,4-diamine | 30 |
| (4-Benzothiazol-2-yl-phenyl)-{6-methoxy-7-[3-(4-methyl-piperazin-1-yl)-propoxy]-quinazolin-4-yl}-amine | 31 |
| (4-Benzothiazol-2-yl-3-methoxy-phenyl)-{6-methoxy-7-[3-(4-methyl-piperazin-1-yl)-propoxy]-quinazolin-4-yl}-amine | 32 |
| (4-Benzothiazol-2-yl-phenyl)-(7H-pyrrolo[2,3-d]pyrimidin-4-yl)-amine | 33 |
| (4-Benzothiazol-2-yl-3-methoxy-phenyl)-(7H-pyrrolo[2,3-d]pyrimidin-4-yl)-amine | 34 |
| (3-Benzothiazol-2-yl-phenyl)-(7H-pyrrolo[2,3-d]pyrimidin-4-yl)-amine | 35 |
| (4-Benzothiazol-2-yl-phenyl)-(2-methoxy-7H-pyrrolo[2,3-d]pyrimidin-4-yl)-amine | 36 |
| (4-Benzothiazol-2-yl-3-methoxy-phenyl)-(2-methoxy-7H-pyrrolo[2,3-d]pyrimidin-4-yl)-amine | 37 |
| (3-Benzothiazol-2-yl-phenyl)-(2-methoxy-7H-pyrrolo[2,3-d]pyrimidin-4-yl)-amine | 38 |
| (4-Benzothiazol-2-yl-phenyl)-{7-methoxy-6-[3-(4-methyl-piperazin-1-yl)-propoxy]-quinazolin-4-yl}-amine | 39 |
| (4-Benzothiazol-2-yl-3-methoxy-phenyl)-{7-methoxy-6-[3-(4-methyl-piperazin-1-yl)-propoxy]-quinazolin-4-yl}-amine | 40 |
| (3-Benzothiazol-2-yl-phenyl)-{7-methoxy-6-[3-(4-methyl-piperazin-1-yl)-propoxy]-quinazolin-4-yl}-amine | 41 |
| (3-Benzothiazol-2-yl-phenyl)-{6-methoxy-7-[3-(4-methyl-piperazin-1-yl)-propoxy]-quinazolin-4-yl}-amine | 42 |
| (4-Benzothiazol-2-yl-3-methoxy-phenyl)-(7H-purin-6-yl)-amine | 43 |
| (4-Benzothiazol-2-yl-2-methyl-phenyl)-{6-methoxy-7-[3-(4-methyl-piperazin-1-yl)-propoxy]-quinazolin-4-yl}-amine | 44 |
| 2-Benzothiazol-2-yl-5-{6-methoxy-7-[3-(4-methyl-piperazin-1-yl)-propoxy]-quinazolin-4-ylamino}-phenol | 45 |
| (5-Benzothiazol-2-yl-2-chloro-phenyl)-{6-methoxy-7-[3-(4-methyl-piperazin-1-yl)-propoxy]-quinazolin-4-yl}-amine | 46 |
| (5-Benzothiazol-2-yl-2-methyl-phenyl)-{6-methoxy-7-[3-(4-methyl-piperazin-1-yl)-propoxy]-quinolin-4-yl}-amine | 47 |
| (4-Benzothiazol-2-yl-2-trifluoromethoxy-phenyl)-{6-methoxy-7-[3-(4-methyl-piperazin-1-yl)-propoxy]-quinazolin-4-yl}-amine | 48 |
| (4-Benzothiazol-2-yl-3-chloro-phenyl)-{6-methoxy-7-[3-(4-methyl-piperazin-1-yl)-propoxy]-quinazolin-4-yl}-amine | 49 |
| (4-Benzothiazol-2-yl-3-fluoro-phenyl)-{6-methoxy-7-[3-(4-methyl-piperazin-1-yl)-propoxy]-quinazolin-4-yl}-amine | 50 |
| (4-Benzothiazol-2-yl-2-methoxy-phenyl)-{6-methoxy-7-[3-(4-methyl-piperazin-1-yl)-propoxy]-quinazolin-4-yl}-amine | 51 |
| (4-Benzothiazol-2-yl-2-fluoro-phenyl)-{6-methoxy-7-[3-(4-methyl-piperazin-1-yl)-propoxy]-quinazolin-4-yl}-amine | 52 |
| 2-Benzothiazol-2-yl-4-{6-methoxy-7-[3-(4-methyl-piperazin-1-yl)-propoxy]-quinazolin-4-ylamino}-phenol | 53 |
| N2-(4-Benzothiazol-2-yl-3-methoxy-phenyl)-N4-(5-rnethyl-1H-pyrazol-3-yl)-pyrimidine-2,4-diamine | 54 |
| N4-(4-Benzothiazol-2-yl-phenyl)-N2-(5-methyl-1H-pyrazol-3-yl)-pyridine-2,4-diamine | 55 |
| 4-(4-Benzothiazol-2-yl-phenylamino)-pyridine-2-carboxylic acid methylamide | 56 |
| (4-Benzothiazol-2-yl-phenyl)-pyridin-4-yl-amine | 57 |
| N4-(4-Benzothiazol-2-yl-phenyl)-N2-methyl-pyridine-2,4-diamine | 58 |
| 5-Benzothiazol-2-yl-2-{6-methoxy-7-[3-(4-methyl-piperazin-1-yl)-propoxy]-quinazolin-4-ylamino}-phenol | 59 |
| {6-Methoxy-7-[3-(4-methyl-piperazin-1-yl)-propoxy]-quinazolin-4-yl}-[4-(5-trifluoromethyl-benzothiazol-2-yl)-phenyl]-amine | 60 |
| (3-Benzothiazol-2-yl-4-chloro-phenyl)-{6-methoxy-7-[3-(4-methyl-piperazin-1-yl)-propoxy]-quinazolin-4-yl}-amine | 61 |
| 2-Benzothiazol-2-yl-6-{6-methoxy-7-[3-(4-methyl-piperazin-1-yl)-propoxy]-quinazolin-4-ylamino}-phenol | 62 |
| (4-Benzothiazol-2-yl-3-methoxy-phenyl)-{6-methoxy-7-[3-(4-methyl-piperazin-1-yl)-propoxy]-quinazolin-4-yl -methyl-amine | 63 |
| (4-Benzothiazol-2-yl-phenyl)-(7-chloro-quinolin-4-yl)-amine | 64 |
| (4-Benzothiazol-2-yl-phenyl)-thieno[3,2-d]pyrimidin-4-yl-amine | 65 |
| (4-Benzothiazol-2-yl-3-methoxy-phenyl)-(7-chloro-quinolin-4-yl)-amine | 66 |
| (4-Benzothiazol-2-yl-3-methoxy-phenyl)-thieno[3,2-d]pyrimidin-4-yl-amine | 67 |
| (3-Benzothiazol-2-yl-phenyl)-(7-chloro-quinolin-4-yl)-amine | 68 |
| (3-Benzothiazol-2-yl-phenyl)-thieno[3,2-d]pyrimidin-4-yl-amine | 69 |
| [4-(5-Chloro-benzothiazol-2-yl)-phenyl]-{6-methoxy-7-[3-(4-methyl-piperazin-1-yl)-propoxy]-quinazolin-4-yl}-amine | 70 |
| (4-Benzothiazol-2-yl-3-methoxy-phenyl)-pyridin-4-yl-amine | 71 |
| (3-Benzothiazol-2-yl-phenyl)-pyridin-4-yl-amine | 72 |
| (4-Benzothiazol-2-yl-3-chloro-phenyl)-(7-chloro-quinolin-4-yl)-amine | 73 |
| (4-Benzothiazol-2-yl-3-chloro-phenyl)-thieno[3,2-d]pyrimidin-4-yl-amine | 74 |
| (4-Benzothiazol-2-yl-2-methyl-phenyl)-{7-methoxy-6-[3-(4-methyl-piperazin-1-yl)-propoxy]-quinazolin- | 75 |
| 2-Benzothiazol-2-yl-5-{7-methoxy-6-[3-(4-methyl-piperazin-1-yl)-propoxy]-quinazolin-4-ylamino}-phenol | 76 |
| (4-Benzothiazol-2-yl-3-chloro-phenyl)-{7-methoxy-6-[3-(4-methyl-piperazin-1-yl)-propoxy]-quinazolin-4-yl}-amine | 77 |
| (4-Benzothiazol-2-yl-3-fluoro-phenyl)-{7-methoxy-6-[3-(4-methyl-piperazin-1-yl)-propoxy]-quinazolin-4-yl}-amine | 78 |
| 5-Benzothiazol-2-yl-2-{7-methoxy-6-[3-(4-methyl-piperazin-1-yl)-propoxy]-quinazolin-4-ylamino}-phenol | 79 |
| (4-Benzothiazol-2-yl-2-methoxy-phenyl)-{7-methoxy-6-[3-(4-methyl-piperazin-1-yl)-propoxy]-quinazolin-4-yl}-amine | 80 |
| (4-Benzothiazol-2-yl-2-fluoro-phenyl)-{7-methoxy-6-[3-(4-methyl-piperazin-1-yl)-propoxy]-quinazolin-4-yl}-amine | 81 |
| {7-Methoxy-6-[3-(4-methyl-piperazin-1-yl)-propoxy]-quinazolin-4-yl}-[4-(5-trifluoromethyl-benzothiazol-2-yl)-phenyl]-amine | 82 |
| [4-(5-Chloro-benzothiazol-2-yl)-phenyl]-{7-methoxy-6-[3-(4-methyl-piperazin-1-yl)-propoxy]-quinazolin-4-yl}-amine | 83 |
| (4-Benzothiazol-2-yl-2-fluoro-phenyl)-(7-chloro-quinolin-4-yl)-amine | 84 |
| (4-Benzothiazol-2-yl-2-fluoro-phenyl)-thieno[3,2-d]pyrimidin-4-yl-amine | 85 |
| N2-(4-Benzothiazol-2-yl-phenyl)-N2-methyl-pyridine-2,4-diamine | 86 |
| (4-Benzothiazol-2-yl-phenyl)-(7-chloro-6-methoxy-quinazolin-4-yl)-amine | 87 |
| [4-(6-Fluoro-benzothiazol-2-yl)-phenyl]-{6-methoxy-7-[3-(4-methyl-piperazin-1-yl)-propoxy]-quinazolin-4-yl}-amine | 88 |
| [4-(6-Chloro-benzothiazol-2-yl)-phenyl]-{6-methoxy-7-[3-(4-methyl-piperazin-1-yl)-propoxy]-quinazolin-4-yl}-amine | 89 |
| [4-(6-Chloro-benzothiazol-2-yl)-2-fluoro-phenyl]-{6-methoxy-7-[3-(4-methyl-piperazin-1-yl)-propoxy]-quinazolin-4-yl}-amine | 90 |
| (4-Benzothiazol-2-yl-3-methyl-phenyl)-{6-methoxy-7-[3-(4-methyl-piperazin-1-yl)-propoxy]-quinazolin-4-yl}-amine | 91 |
| [4-(6-Methoxy-benzothiazol-2-yl)-phenyl]-{6-methoxy-7-[3-(4-methyl-piperazin-1-yl)-propoxy]-quinazolin-4-yl}-amine | 92 |
| 4-(4-Benzothiazol-2-yl-phenoxy)-6-methoxy-7-[3-(4-methyl-piperazin-1-yl)-propoxy]-quinazoline | 93 |
| 4-(4-Benzothiazol-2-yl-2-fluoro-phenoxy)-6-methoxy-7-[3-(4-methyl-piperazin-1-yl)-propoxy]-quinazoline | 94 |

The compounds of the formula (I) to be used according to the invention can form salts with inorganic or organic acids or bases. Examples of pharmaceutically acceptable salts comprise without limitation non-toxic inorganic or organic salts such as acetate derived from acetic acid, aconitate derived from aconitic acid, ascorbate derived from ascorbic acid, benzoate derived from benzoic acid, cinnamate derived from cinnamic acid, citrate derived from citric acid, embonate derived from embonic acid, enantate derived from heptanoic acid, formiate derived from formic acid, fumarate derived from fumaric acid, glutamate derived from glutamic acid, glycolate derived from glycolic acid, chloride derived from hydrochloric acid, bromide derived from hydrobromic acid, lactate derived from lactic acid, maleate derived from maleic acid, malonate derived from malonic acid, mandelate derived from mandelic acid, methanesulfonate derived from methanesulfonic acid, naphtaline-2-sulfonate derived from naphtaline-2-sulfonic acid, nitrate derived from nitric acid, perchlorate derived from perchloric acid, phosphate derived from phosphoric acid, phthalate derived from phthalic acid, salicylate derived from salicylic acid, sorbate derived from sorbic acid, stearate derived from stearic acid, succinate derived from succinic acid, sulphate derived from sulphuric acid, tartrate derived from tartaric acid, toluene-*p*-sulfate derived from *p*-toluenesulfonic acid and others. Such salts can be produced by methods known to someone of skill in the art and described in the prior art.

Other salts like oxalate derived from oxalic acid, which is not considered as pharmaceutically acceptable can be appropriate as intermediates for the production of compounds of the formula (I, II, III, IV) or a pharmaceutically acceptable salt thereof or physiologically functional derivative or a stereoisomer thereof.

The compounds according to the invention and medicaments prepared therewith are generally useful for the treatment of cell proliferation disorders, for the treatment or prophylaxis of immunological diseases and conditions (as for instance inflammatory diseases, neuroimmunological diseases, autoimmune diseases or other).
The compounds of the present invention are useful for the treatment of diseases which are caused by malignant cell proliferation, such as all forms of solid tumors, leukemias and lymphomas. Therefore the compounds according to the invention and medicaments prepared therewith are generally useful for regulating cell activation, cell proliferation, cell survival, cell differentiation, cell cycle, cell maturation and cell death or to induce systemic changes in metabolism such as changes in sugar, lipid or protein metabolism. They can also be used to support cell generation poiesis, including blood cell growth and generation (prohematopoietic effect) after depletion or destruction of cells, as caused by, for example, toxic agents, radiation, immunotherapy, growth defects, malnutrition, malabsorption, immune dysregulation, anemia and the like or to provide a therapeutic control of tissue generation and degradation, and therapeutic modification of cell and tissue maintenance and blood cell homeostasis.

These diseases and conditions include but are not limited to cancer as hematological (e.g. leukemia, lymphoma, myeloma) or solid tumors (for example breast, prostate, liver, bladder, lung, esophageal, stomach, colorectal, genitourinary, gastrointestinal, skin, pancreatic, brain, uterine, colon, head and neck, cervical, and ovarian, melanoma, astrocytoma, small cell lung cancer, glioma, basal and squameous cell carcinoma, sarcomas as Kaposi's sarcoma and osteosarcoma).

Other aspects of the present invention relate to 2-arylbenzothiazole derivatives of the general formula (I, II, III, IV) as new pharmaceutically active agents, especially for the preparation of a pharmaceutical composition for the treatment of diseases which are cured or relieved by the inhibition of one or several kinases and/or phosphatases.

In one embodiment, the compounds of the formula (I, II, III, IV) may be used for treating, relieving, and/or preventing diseases in which cell proliferation disorders play a role.

In another embodiment, the compounds of formula (I, II, III, IV) may be used for treating, relieving, and/or preventing cancer.

In another preferred embodiment of the invention the compounds of formula (I, II, III, IV) may be used for treating, relieving, and/or preventing all forms of solid tumors (including, but not limited to, the following: breast, prostate, liver, bladder, lung, esophageal, stomach, colorectal, genitourinary, gastrointestinal, skin, pancreatic, brain, uterine, colon, head and neck, cervical, and ovarian, small cell lung cancer, melanoma, astrocytoma, glioma, basal and squameous cell carcinoma, sarcomas as Kaposi's sarcoma and osteosarcoma).

In a more preferred embodiment of the invention the compounds of formula (I, II, III, IV) may be used for treating, relieving, and/or preventing diseases by inhibition of one or more protein kinases.

In another more preferred embodiment of the invention the compounds of formula (I, II, III, IV) may be used for treating and/or preventing diseases by inhibition of one or or more kinases like: Aurora-A, Aurora-B, EGF-R, ERBB2, PDGFR, IGF1-R, VEGF-R2, VEGF-R3, EPHB4, TIE2, and SRC.

In one embodiment, the compounds of the formula (I, II, III, IV) may be used for treating and/or preventing diseases in which T cells play a role, especially inflammatory disorders and immune disorders such as Addison's disease, alopecia areata, Ankylosing spondylitis, haemolytic anemia (anemia haemolytica), pernicious anemia (anemia perniciosa), aphthae, aphthous stomatitis, arthritis, arteriosclerotic disorders, osteoarthritis, rheumatoid arthritis, aspermiogenese, asthma bronchiale, auto-immune asthma, auto-immune hemolysis, Bechet's disease, Boeck's disease, inflammatory bowel disease, Burkitt's lymphoma, Crohn's disease, chorioiditis, colitis ulcerosa, Coeliac disease, cryoglobulinemia, dermatitis herpetiformis, dermatomyositis, insulin-dependent type I diabetes, juvenile diabetes, idiopathic diabetes insipidus, insulin-dependent diabetes mellisis, autoimmune demyelinating diseases, Dupuytren's contracture, encephalomyelitis, encephalomyelitis allergica, endophthalmia phacoanaphylactica, enteritis allergica, autoimmune enteropathy syndrome, erythema nodosum leprosum, idiopathic facial paralysis, chronic fatigue syndrome, febris rheumatica, glomerulo nephritis, Goodpasture's syndrome, Graves' disease, Harnman-Rich's disease, Hashimoto's disease, Hashimoto's thyroiditis, sudden hearing loss, sensoneural hearing loss, hepatitis chronica, Hodgkin's disease, haemoglobinuria paroxysmatica, hypogonadism, ileitis regionalis, iritis, leucopenia, leucemia, lupus erythematosus disseminatus, systemic lupus erythematosus, cutaneous lupus erythematosus, lymphogranuloma malignum, mononucleosis infectiosa, myasthenia gravis, traverse myelitis, primary idiopathic myxedema, nephrosis, ophthalmia symphatica, orchitis granulomatosa, pancreatitis, pemphigus, pemphigus vulgaris, polyarteritis nodosa, polyarthritis chronica primaria, polymyositis, polyradiculitis acuta, psoriasis, purpura, pyoderma gangrenosum, Quervain's thyreoiditis, Reiter's syndrome, sarcoidosis, ataxic sclerosis, progressive systemic sclerosis, scleritis, sclerodermia, multiple sclerosis, sclerosis disseminata, acquired spenic atrophy, infertility due to antispermatozoan antibodies, thrombocytopenia, idiopathic thrombocytopenia purpura, thymoma, acute anterior uveitis, vitiligo, AIDS, HIV, SCID and Epstein Barr virus associated diseases such as Sjorgren's syndrome, virus (AIDS or EBV) associated B cell lymphoma, parasitic diseases such as Leishmania, and immunesuppressed disease states such as viral infections following allograft transplantations, AIDS, cancer, chronic active hepatitis diabetes, toxic chock syndrome and food poisoning.

Thus, in one embodiment, the invention relates to the use of the compounds of the formula (I, II, III, IV) or a pharmaceutically acceptable salt or physiologically functional derivative or a stereoisomer thereof if desired with appropriate adjuvants and additives for the production of a medicament for the treatment or prevention of a disease characterized by hyperproliferation of keratinocytes and/or T cells, especially inflammatory disorders and immune disorders, preferably selected from the group consisting of Addison's disease, alopecia areata, Ankylosing spondylitis, haemolytic anemia (anemia haemolytica), pernicious anemia (anemia perniciosa), aphthae, aphthous stomatitis, arthritis, arteriosclerotic disorders, osteoarthritis, rheumatoid arthritis, aspermiogenese, asthma bronchiale, auto-immune asthma, auto-immune hemolysis, Bechet's disease, Boeck's disease, inflammatory bowel disease, Burkitt's lymphoma, Crohn's disease, chorioiditis, colitis ulcerosa, Coeliac disease, cryoglobulinemia, dermatitis herpetiformis, dermatomyositis, insulin-dependent type I diabetes, juvenile diabetes, idiopathic diabetes insipidus, insulin-dependent diabetes mellisis, autoimmune demyelinating diseases, Dupuytren's contracture, encephalomyelitis, encephalomyelitis allergica, endophthalmia phacoanaphylactica, enteritis allergica, autoimmune enteropathy syndrome, erythema nodosum leprosum, idiopathic facial paralysis, chronic fatigue syndrome, febris rheumatica, glomerulo nephritis, Goodpasture's syndrome, Graves' disease, Harnman-Rich's disease, Hashimoto's disease, Hashimoto's thyroiditis, sudden hearing loss, sensoneural hearing loss, hepatitis chronica, Hodgkin's disease, haemoglobinuria paroxysmatica, hypogonadism, ileitis regionalis, iritis, leucopenia, leucemia, lupus erythematosus disseminatus, systemic lupus erythematosus, cutaneous lupus erythematosus, lymphogranuloma malignum, mononucleosis infectiosa, myasthenia gravis, traverse myelitis, primary idiopathic myxedema, nephrosis, ophthalmia symphatica, orchitis granulomatosa, pancreatitis, pemphigus, pemphigus vulgaris, polyarteritis nodosa, polyarthritis chronica primaria, polymyositis, polyradiculitis acuta, psoriasis, purpura, pyoderma gangrenosum, Quervain's thyreoiditis, Reiter's syndrome, sarcoidosis, ataxic sclerosis, progressive systemic sclerosis, scleritis, sclerodermia, multiple sclerosis, sclerosis disseminata, acquired spenic atrophy, infertility due to antispermatozoan antibodies, thrombocytopenia, idiopathic thrombocytopenia purpura, thymoma, acute anterior uveitis, vitiligo, AIDS, HIV, SCID and Epstein Barr virus associated diseases such as Sjorgren's syndrome, virus (AIDS or EBV) associated B cell lymphoma, parasitic diseases such as Leishmania, and immunesuppressed disease states such as viral infections following allograft transplantations, AIDS, cancer, chronic active hepatitis diabetes, toxic chock syndrome and food poisoning.

"Treatment" according to the present invention is intended to mean complete or partial healing of a disease, or alleviation of a disease or stop of progression of a given disease.

The compounds of the present invention can further be used for diseases that are caused by protozoal infestations in humans and animals. Such veterinary and human pathogenic protozoas are preferably intracellular active parasites of the phylum Apicomplexa or Sarcomastigophora, especially Trypanosoma, Plasmodia, Leishmania, Babesia and Theileria, Cryptosporidia, Sacrocystida, Amoebia, Coccidia and Trichomonadia. These active substances or corresponding drugs are especially suitable for the treatment of Malaria tropica, caused by *Plasmodium falciparum,* Malaria tertiana, caused by *Plasmodium vivax* or *Plasmodium ovale* and for the treatment of Malaria quartana, caused by *Plasmodium malariae.* They are also suitable for the treatment of Toxoplasmosis, caused by *Toxoplasma gondii,* Coccidiosis, caused for instance by *Isospora belli,* intestinal Sarcosporidiosis, caused by *Sarcocystis suihominis,* dysentery caused by *Entamoeba histolytica,* Cryptosporidiosis, caused by *Cryptosporidium parvum,* Chargas' disease, caused by *Trypanosoma cruzi,* sleeping sickness, caused by *Trypanosoma brucei rhodesiense* or *gambiense,* the cutaneous and visceral as well as other forms of Leishmaniosis. They are also suitable for the treatment of animals infected by veterinary pathogenic protozoa, like *Theileria parva,* the pathogen causing bovine East coast fever, *Trypanosoma congolense congolense* or *Trypanosoma vivax vivax, Trypanosoma brucei brucei,* pathogens causing Nagana cattle disease in Africa, *Trypanosoma brucei evansi* causing Surra, *Babesia bigemina,* the pathogen causing Texas fever in cattle and buffalos, *Babesia bovis,* the pathogen causing European bovine Babesiosis as well as Babesiosis in dogs, cats and sheep, *Sarcocystis ovicanis* and *ovifelis* pathogens causing Sarcocystiosis in sheep, cattle and pigs, Cryptosporidia, pathogens causing Cryptosporidioses in cattle and birds, Eimeria and Isospora species, pathogens causing Coccidiosis in rabbits, cattle, sheep, goats, pigs and birds, especially in chickens and turkeys. The use of the compounds of the present invention is preferred in particular for the treatment of Coccidiosis or Malaria infections, or for the preparation of a drug or feed stuff for the treatment of these diseases. This treatment can be prophylactic or curative. In the treatment of malaria, the compounds of the present invention may be combined with other anti-malaria agents.

The compounds of the present invention can further be used for viral infections or other infections caused for instance by *Pneumocystis carinii.*

Furthermore, the invention relates to a method of treatment or prevention of diseases which comprises the administration of an effective amount of compounds of the formula (I, II, III, IV) or a pharmaceutically acceptable salt or physiologically functional derivative or a stereoisomer thereof.

The compounds of formula (I, II, III, IV) and their pharmacologically acceptable salts can be administered to animals, preferably to mammals, and in particular to humans, dogs and chickens as therapeutics per se, as mixtures with one another or in the form of pharmaceutical preparations which allow enteral or parenteral use and which as active constituent contain an effective dose of at least one compound of the formula (I, II, III, IV) or a salt thereof, in addition to customary pharmaceutically innocuous excipients and additives. The compounds of formula (I, II, III, IV) can also be administered in form of their salts, which are obtainable by reacting the respective compounds with physiologically acceptable acids and bases.

The production of medicaments containing the compounds of formula (I, II, III, IV) according to the invention and their application can be performed according to well-known pharmaceutical methods.

While the compounds of formula (I, II, III, IV) according to the invention for use in therapy may be administered in the form of the raw chemical compound, it is preferred to introduce the active ingredient, optionally in the form of a physiologically acceptable salt in a pharmaceutical composition together with one or more adjuvants, excipients, carriers, buffers, diluents, and/or other customary pharmaceutical auxiliaries. Such salts of the compounds may be anhydrous or solvated.

In a preferred embodiment, the invention provides medicaments comprising compounds of formula (I, II, III, IV) according to the invention, or a pharmaceutically acceptable salt or physiologically functional derivative or a stereoisomer thereof, together with one or more pharmaceutically acceptable carriers thereof, and, optionally, other therapeutic and/or prophylactic ingredients. The carrier(s) must be "acceptable" in the sense of being compatible with the other ingredients of the formulation and not harmful to the recipient thereof.

A medicament of the invention may be those suitable for oral, rectal, bronchial, nasal, topical, buccal, sub-lingual, transdermal, vaginal or parenteral (including cutaneous, subcutaneous, intramuscular, intraperitoneal, intravenous, intraarterial, intracerebral, intraocular injection or infusion) administration, or those in a form suitable for administration by inhalation or insufflation, including powders and liquid aerosol administration, or by sustained release systems. Suitable examples of sustained release systems include semipermeable matrices of solid hydrophobic polymers containing the compound of the invention, which matrices may be in form of shaped articles, e.g. films or microcapsules.

The compounds according to the invention, together with a conventional adjuvant, carrier, or diluent, may thus be placed into the form of medicament and unit dosages thereof. Such forms include solids, and in particular tablets, filled capsules, powder and pellet forms, and liquids, in particular aqueous or non-aqueous solutions, suspensions, emulsions, elixirs, and capsules filled with the same, all for oral use, suppositories for rectal administration, and sterile injectable solutions for parenteral use. Such Medicament and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

The compound useable according to the invention can be administered in a wide variety of oral and parenteral dosage forms. It will be obvious to those skilled in the art that the following dosage forms may comprise, as the active component, either a compound of formula (I, II, III, IV) according to the invention or a pharmaceutically acceptable salt or stereosomer thereof.

For preparing a medicament from a compounds of formula (I, II, III, IV) pharmaceutically acceptable carriers can be either solid or liquid. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid which is in a mixture with the finely divided active component. In tablets, the active component is mixed with the carrier having the necessary binding capacity in suitable proportions and compacted in the shape and size desired. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify. Compositions suitable for vaginal administration may be presented as pessaries, tampons, creams, gels, pastes, foams or sprays containing in addition to the active ingredient such carriers as are known in the art to be appropriate. Liquid preparations include solutions, suspensions, and emulsions, for example, water or water-propylene glycol solutions. For example, parenteral injection liquid preparations can be formulated as solutions in aqueous polyethylene glycol solution.

The compounds of formula (I, II, III, IV) according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative.

The compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.
Aqueous solutions suitable for oral use can be prepared by dissolving the active component in water and adding suitable colorants, flavours, stabilising and thickening agents, as desired. Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavours, stabilisers, buffers, artificial and natural sweeteners, dispersants, thickeners, solubilizing agents, and the like.

In one embodiment of the present invention, the medicament is applied topically or systemically or via a combination of the two routes.

In an especially preferred embodiment of the present invention the medicament is applied topically. This reduces possible side effects and limits the necessary treatment to those areas affected.

Preferably the medicament is prepared in form of an ointment, a gel, a plaster, an emulsion, a lotion, a foam, a cream of a mixed phase or amphiphilic emulsion system (oil/water-water/oil mixed phase), a liposome, a transfersome, a paste or a powder.

Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

Compositions suitable for topical administration in the mouth include lozenges comprising the active agent in a flavoured base, usually sucrose and acacia or tragacanth; pastilles comprising the active ingredient in an inert base such as gelatin and glycerine or sucrose and acacia; and mouthwashes comprising the active ingredient in a suitable liquid carrier.

Solutions or suspensions are applied directly to the nasal cavity by conventional means, for example with a dropper, pipette or spray. The compositions may be provided in single or multi-dose form. In the latter case of a dropper or pipette, this may be achieved by the patient administering an appropriate, predetermined volume of the solution or suspension. In the case of a spray, this may be achieved for example by means of a metering atomising spray pump.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity The powder composition may be presented in unit dose form for example in capsules or cartridges of, e.g., gelatin, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronization.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these in packaged form. Tablets or capsules for oral administration and liquids for intravenous administration and continuous infusion are preferred compositions.

Further details on techniques for formulation and administration may be found in the latest edition of Remington's Pharmaceutical Sciences (Maack Publishing Co. Easton, Pa.).

Pharmaceutical compositions can also contain two or more compounds of the formula (I, II, III, IV) or their pharmacologically acceptable salts and also other therapeutically active substances.

Thus, the compounds of the present invention can be used in the form of one compound alone or in combination with other active compounds - for example with medicaments already known for the treatment of the aforementioned diseases, whereby in the latter case a favorable additive, amplifying effect is noticed. Suitable amounts to be administered to humans range from 5 to 500 mg.

To prepare the pharmaceutical preparations, pharmaceutically inert inorganic or organic excipients can be used. To prepare pills, tablets, coated tablets and hard gelatin capsules, for example, lactose, corn starch or derivatives thereof, talc, stearic acid or its salts, etc. can be used. Excipients for soft gelatin capsules and suppositories are, for example, fats, waxes, semi-solid and liquid polyols, natural or hardened oils etc. Suitable excipients for the production of solutions and syrups are, for example, water, sucrose, invert sugar, glucose, polyols etc. Suitable excipients for the production of injection solutions are, for example, water, alcohols, glycerol, polyols or vegetable oils.

The dose can vary within wide limits and is to be suited to the individual conditions in each individual case. For the above uses the appropriate dosage will vary depending on the mode of administration, the particular condition to be treated and the effect desired. In general, however, satisfactory results are achieved at dosage rates of about 1 to 100 mg/kg animal body weight preferably 1 to 50 mg/kg. Suitable dosage rates for larger mammals, for example humans, are of the order of from about 10 mg to 3 g/day, conveniently administered once, in divided doses 2 to 4 times a day, or in sustained release form.

In general, a daily dose of approximately 10 mg to 5000 mg, preferably 50 to 500 mg, per human individual is appropriate in the case of the oral administration. In the case of other administration forms too, the daily dose is in similar ranges. For topical delivery, depending on the permeability of the skin, the type and the severity of the disease and dependent on the type of formulation and frequency of application, different concentrations of active compounds within the medicament can be sufficient to elicit a therapeutic effect by topical application. Preferably the concentration of an active compound or a pharmaceutically acceptable salt thereof or a physiologically functional derivative or a stereoisomer thereof within a medicament according to the invention is in the range of between 1 µmol/l and 100 mmol/l.

The following examples and figures are included to demonstrate preferred embodiments of the invention. It should be appreciated by those of skill in the art that the techniques disclosed in the examples that follow represent techniques discovered by the inventors to function well in the practice of the invention, and thus can be considered preferred modes for its practice. However, those of skill in the art should, in light of the present disclosure, appreciate that many changes can be made in the specific embodiments that are disclosed without departing from the spirit and scope of the invention as set out in the appended claims. All references cited are incorporated herein by reference.

### Examples

Abbreviations: min, minute(s); h, hour(s); r.t., room temperature; TLC, thin layer chromatography; XANTPHOS, 9,9-dimethyl-4,5-bis(diphenylphosphino)xanthene; dba, dibenzylideneacetone; DME, 1,2-dimethoxyethane; DIEA, *N,N*-diisopropylethylamine; EDC, 1-(3-dimethylaminopropyl)-3-ethylcarbodiimide.

Preparative HPLC-MS: Waters 600 Multisolvent Delivery System with peparative pump heads. 2000 µl or 5000 µl Sample loop. Column, Waters X-Terra RP18, 7 µm, 19 x 150 mm with X-Terra RP 18 guard cartridge 7 µm, 19 x 10 mm; used at flow rate 20 ml/min or YMC ODS-A, 120 Å, 40 x 150 mm with X-Terra RP18 guard cartridge 7 µm, 19 x 10 mm; used at flow rate 50 ml/min. Make-up solvent: MeCN - H₂O - HCO₂H 80 : 20 : 0.05 (v:v:v). Eluent A, H₂O + 0. 1 % HCO₂H; eluent B, MeCN. Different linear gradients from 5 - 100% eluent B, adapted to sample. Injection volume: 500 µl - 2000 µl depending on sample.

### Syntheses of Intermediates.

### General procedure 1:

**Syntheses of 4-chloroquinazolines with alkylamino sidechains:** 4-Chloro-6-methoxy-7-[3-(4-methylpiperazin-1-yl)propoxy]quinazoline, 4-Chloro-7-methoxy-6-[3-(4-methylpiperazin-1-yl)propoxy]quinazoline and 4-Chloro-6-methoxy-7-(3-pyrrolidin-1-yl-propoxy)-quinazoline.

Step 1. To a solution of methyl vanillate or methyl isovanillate (7.29 g, 40 mmol) in dimethylformamide (25 mL), potassium carbonate (8.29 g, 60 mmol) and benzyl bromide (5.26 mL, 44 mmol) were added. The mixture was heated to 100°C for 3 h. After cooling to r.t., water was added and the product was extracted several times with ethyl acetate. The combined organic phases were washed with water and brine. After drying over Na₂SO₄, the solvent was removed to yield methyl 4-benzyloxy-3-methoxybenzoate or methyl 3-benzyloxy-4-methoxybenzoate, respectively, quantitatively, which was used without further purification.

Step 2. Crude material of step 1 (40.0 mmol) was converted into methyl 4-benzyloxy-5-methoxy-2-nitrobenzoate or methyl 5-benzyloxy-4-methoxy-2-nitrobenzoate, respectively, in 91-94% yield as described in US 02/0026052 A1, page 51, reference example 15.

Step 3. In a 1 1 Schlenk flask filled with argon, product of step 2 (36.6 mmol) and palladium on charcoal (1.17 g, 10% Pd, 1.1 mmol Pd) were combined and tetrahydrofuran (250 mL) was added. The argon was replaced with hydrogen (1 bar), and the mixture was vigorously stirred at r.t. until completion of the reaction. The palladium was separated by filtration through a pad of celite and the solvent was removed to obtain methyl 2-amino-4-hydroxy-5-methoxybenzoate or methyl 2-amino-5-hydroxy-4-methoxybenzoate, respectively, quantitatively, which, again, was used without further purification.

Step 4. A mixture of formamide (29 mL), ammonium formate (3.41 g, 54 mmol) and crude material of step 3 (36.0 mmol) was heated to 140°C for 4 h. After cooling to r.t., water (75 mL) was added. After stirring for 1 h, the precipitated 7-hydroxy-6-methoxy-3,4-dihydroquinazolin-4-one or 6-hydroxy-7-methoxy-3,4-dihydroquinazolin-4-one, respectively, was filtered off, washed with water and dried (76-85%).

Step 5. A mixture of product step 4 (30.5 mmol), acetic anhydride (21.5 mL, 229 mmol) and pyridine (4.9 mL, 61 mmol) was heated to 100°C for 4 h. After cooling to r.t., ice water (200 mL) was added and the mixture was vigorously stirred for 1 h. The precipitated 7-acetoxy-6-methoxy-3,4-dihydroquinazolin-4-one or 6-acetoxy-7-methoxy-3,4-dihydroquinazolin-4-one, respectively, was filtered off, washed with water and dried (93-96%).

Step 6. Product step 5 (8.54 mmol) was converted into 4-chloro-7-hydroxy-6-methoxyquinazoline or 4-chloro-6-hydroxy-7-methoxyquinazoline, respectively, (58-95%) by reacting them with thionylchloride (12 mL) and DMF (0.3 mL) at 85 °C for 1.5 h. Excess thionylchloride was removed by distillation. Traces of thionylchloride were removed by aceotropic distillation wit toluene (two times). Alternatively the products step 5 can be converted into the chlorides by reacting them with a mixture of POCl₃ and PCl₅. The acetyl groups were removed by hydrolysis with ammonium hydroxide (5 mL, 28-30 wt%) in dioxane / water (100 mL / 20 mL) at 0 °C to r.t.

Step 7.

### General procedure 1:

Di-*tert*-butyl azodicarboxylate (0.478 g, 2.08 mmol) was added portionwise to a mixture of product step 6 (1.66 mmol), 3-(4-methylpiperazin-1-yl)-propan-1-ol (synthesis described below, 0.276 g, 1.74 mmol), and triphenylphosphine (0.544 g, 2.08 mmol) in dichloromethane (20 mL) at r.t.. If necessary, further alcohol was added. After stirring for 2 h, the solution was concentrated to 10 mL, mounted on silica and chromatographed (gradient, dichloromethane to dichloromethane : methanol = 3:2) to obtain the desired ethers (~73%).

Synthesis of 4-chloro-6-methoxy-7-[3-(4-methylpiperazin-1-yl)propoxy]quinazoline:

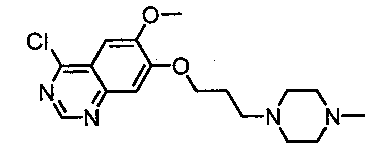

The compound was synthesised according to general procedure 1 from 4-chloro-7-hydroxy-6-methoxyquinazoline. LC/ESI-MS: *m*/*z* = 351 [M+H].

Synthesis of 4-chloro-7-methoxy-6-[3-(4-methylpiperazin-1-yl)propoxy]quinazoline:

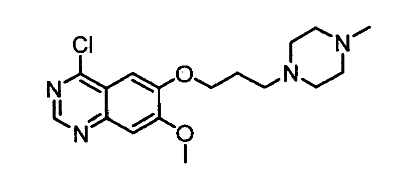

The compound was synthesised according to general procedure 1 from 4-chloro-6-hydroxy-7-methoxyquinazoline. LC/ESI-MS: *m*/*z* = 351 [M+H].

Synthesis of 4-chloro-6-methoxy-7-(3-pyrrolidin-1-yl-propoxy)-quinazoline:

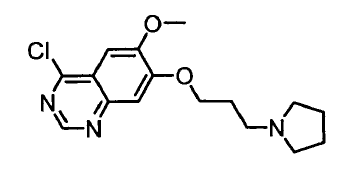

The compound was synthesised according to general procedure 1 from 4-chloro-7-hydroxy-6-methoxyquinazoline. LC/ESI-MS: *mlz* = 322 [M+H].

### Synthesis of 3-(4-methylpiperazin-1-yl)-propan-1-ol:

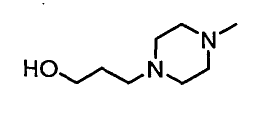

1-Methylpiperazine (6.99 mL, 63 mol) was dissolved in toluene (30 mL). 3-Bromopropanol (2.62 mL, 30 mmol) was added slowly and the mixture was stirred overnight at r.t.. After heating to 80°C for 2 h and cooling to r.t., the mixture was filtered and the filter cake was thoroughly washed with toluene. After removal of the solvent, the residue was subjected to Kugelrohr distillation (b.p., 180°C / 2 mbar) to obtain a colourless oil (4.08 g, 25.8 mmol, 86%). LC/ESI-MS: *m*/*z =* 159 [M+H].

### Synthesis of 3-morpholin-4-yl-propan-1-ol:

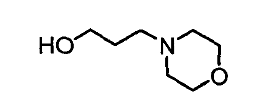

3-Morpholin-4-yl-propan-1-ol (b.p., 180°C / 1 mbar) was synthesized in the same manner as 3-(4-methylpiperazin-1-yl)-propan-1-ol from 3-bromopropanol and morpholine. LC/ESI-MS: *m*/*z* = 146 [M+H].

### Synthesis of 3-Pyrrolidin-1-yl-propan-1-ol:

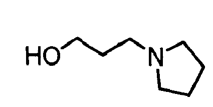

3-Pyrrolidin-1-yl-propan-1-ol (b.p., 230°C / 10 mbar) was synthesized in the same manner as 3-(4-methylpiperazin-1-yl)-propan-1-ol from 3-bromopropanol and pyrrolidine. LC/ESI-MS: *m*/*z =* 130 [M+H].

### Synthesis of 2-(4-Methyl-piperazin-1-yl)-ethanol:

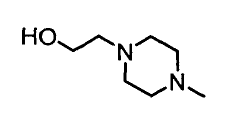

2-(4-Methyl-piperazin-1-yl)-ethanol (b.p., 115-135°C / 0.1 mbar) was synthesized according to the synthesis of 3-(4-methylpiperazin-1-yl)-propan-1-ol from 2-bromo-ethanol and 1-methylpiperazine. LC/ESI-MS: *m*/*z =* 145 [M+H].

### Synthesis of 2-morpholin-4-yl-ethanol:

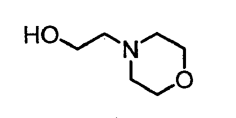

2-Morpholin-4-yl-ethanol (b.p., 130-145°C / 20 mbar) was synthesized according to the synthesis of 3-(4-methylpiperazin-1-yl)-propan-1-ol from 2-bromo-ethanol and morpholine. LC/ESI-MS: *m*/*z =* 132 [M+H].

**Synthesis of differently substituted 2- or 4-chloropyrimidines:** 2-Chloro-4-(4-methylpiperazinl-yl)pyrimidine, (2-chloropyrimidin-4-yl)-(5-methyl-*1H*-pyrazol-3-yl)-amine, (2-chloropyrimidin-4-yl)-methylamine, (4-chloropyrimidin-2-yl)-methylamine. Syntheses were performed in analogy to T. Kumagai et al., Bioorg. Med. Chem. 2001, 9, 1349-1355; S. F. Campbell et al., J. Med. Chem. 1987, 30, 1794-1798; US 2004/0132781 A1, [0851].

A mixture of 2,4-dichloropyrimidine (0.967 g, 6.49 mmol), the respective amine 1-methylpiperazine (0.65 g, 6.49 mmol), 3-amino-5-methylpyrazole (0.63 g, 6.49 mmol) or methylamine hydrochloride (0.44 g, 6.49 mmol), and ethyldiisopropylamine (2.83 mL, 16.22 mmol; 3.96 mL, 22.71 mmol in case of methylamine hydrochloride) in ethanol (13 mL) was stirred at -10°C for 2 h and then at r.t. overnight. For weaker nucleophiles like aminopyrazoles, the mixture had to be stirred at 50°C for 4 h additionally to get complete conversion.

Mixtures were partitioned between H₂O/brine (3:1; 100 mL) and chloroform (3 x 70 mL). Combined organic phases were washed once with brine (50 mL) and dried over MgSO₄.

2-Chloro-4-(4-methylpiperazinl-yl)pyrimidine:

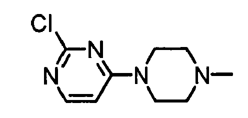

Removal of solvent yielded a pale-beige solid, which was washed with ethyl acetate / ultrasound to give the desired product as a colourless powder, which was further washed with Et₂O. Additional product was obtained upon fractional crystallization of the washing solution. A total of 0.741 g (3.48 mmol, 54%) of 2-chloro-4-(4-methylpiperazinl-yl)-pyrimidine was obtained.

(2-Chloropyrimidin-4-yl)-(5-methylpyrazol-3-yl)amine:

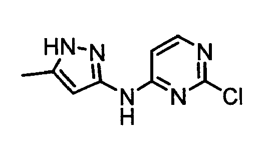

Upon fractional crystallization from chloroform/diethylether, (2-chloropyrimidin-4-yl)-(5-methylpyrazol-3-yl)amine (0.258 g, 1.23 mmol, 19%) was obtained as colourless crystals.

(2-Chloropyrimidin-4-yl)-methylamine 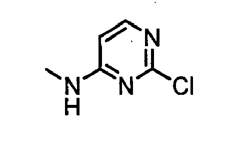 and (4-chloropyrimidin-2-yl)-methylamine:

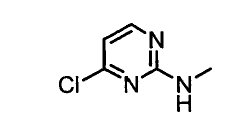

Fractional crystallization from ethyl acetate/diethylether yielded (2-chloropyrimidin-4-yl)-methylamine as the major product within first fractions and enriched (4-chloropyrimidin-2-yl)-methylamine as minor product in the latter fractions, each as colorless powder. A total of 0.396 g (2.57 mmol, 40%) of (2-chloropyrimidin-4-yl)-methylamine and 0.118 g (0.822 mmol, 13%) of (4-chloropyrimidin-2-yl)-methylamine was attained.

### General procedure 2:

**Synthesis of 3- and 4-(benzothiazol-2-yl)-phenylamines and -phenols from 2-aminothiophenols** (according to I. Hutchinson et al., J. Med. Chem. 2001, 44, 1446-1455): Differently substituted 2-aminothiophenols (1.1 equiv.) and 3- or 4-aminobenzoic acids /3- or 4-hydroxybenzoic acids (1.0 equiv.), respectively, were placed in a flask and treated with polyphosphoric acid (1.0 g per 1.0 mmol aminobenzoic acid) at 185°C for 5 h. For aminobenzoic acids additionally substituted with free phenolic OH-groups, 3.0 equiv. of 2-aminothiophenols had to be used.
Still hot, the mixture was neutralized by pouring it into icy NaOH solution (1 mmol NaOH per 1.0 g polyphosphoric acid, dissolved in 10 mL H₂O and mixed with ice). A precipitation formed and still remaining polyphosphoric acid (brown-black gummy slurry) was dissolved by further addition of 5% aq. NaOH and ultrasonic treatment. The precipitate was filtered off and washed thoroughly with 5% aq. NaOH and H₂O. If precipitate was too fine for filtering, a centrifuge was used for sedimentation of the solid. Product was next dissolved in 400 mL DMF/MeOH (1:1) at 60°C, cooled down to r.t. and precipitated by adding H₂O. Product was filtered off (frit 3 and subsequently, frit 4) and dried in high vacuum.
If direct precipitation did not succeed properly upon pouring into icy NaOH, the aq. slurry was extracted with CHCl₃/ethyl acetate 1:1 (3 x). Combined org. phases were dried over MgSO₄ and, if necessary, purified using chromatography on silica gel, solvent: petroleum ether / ethyl acetate 10:1 to 1:3.

### General procedure 3:

**Synthesis of substituted 3- and 4-(benzothiazol-2-yl)-phenylamines from 2-substituted aminobenzothiazoles** (according to I. Hutchinson et al., J. Med. Chem. 2001, 44, 1446-1455):
Differently substituted 2-amino- or 2-methylbenzothiazoles (3 mmol) were added to a solution of potassium hydroxide (2.5 g) in water (5 mL). The resulting mixture was heated under reflux for 5 h, after which complete solution had occurred. After cooling, the reaction mixture was acidified (to pH 6) by the addition of acetic acid. Water (50 mL) was added, and the resulting mixture was stirred overnight. The solid precipitate was collected and purified by column chromatography (CH₂Cl₂) to give the corresponding bis(2-aminoaryl)disulfide.
To a solution of the disulfide (1 mmol) in pyridine (5 mL), 4-nitrobenzoyl chloride (2 mmol) or the corresponding derivative was added. The resulting mixture was heated under reflux for 30 min and then poured into water (15 mL). The precipitate was collected, washed with water (20 mL) and purified by column chromatography (CH₂Cl₂).
The precipitate was reductively cyclized as follows: To a solution of 10 M aq. HCl (10 mL) and ethanol/H₂O 10:1 (22 mL) was added the disulfide (1.6 mmol) and tin(II) chloride dihydrate (9.8 mmol). The reaction mixture was heated under reflux for 15 h, cooled to 25 °C, and poured into water (75 mL). Sodium hydroxide (2 g) was added slowly, and the mixture was stirred for 1 h. The precipitate was collected, washed with water (10 mL) and purified by column chromatography (CH₂Cl₂).

### Synthesis of 2-(4-bromophenyl)-benzothiazole:

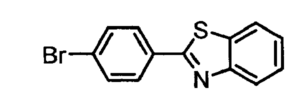

Preparation was performed according to the general procedure 2 described above for 3-and 4-(benzothiazol-2-yl)-phenylamines and -phenols from 2-aminothiophenols and, in this case, 4-bromobenzoic acid. LC/ESI-MS: *m*/*z* = 290 [M+H].

### Synthesis of 4-benzothiazol-2-yl-phenylamine:

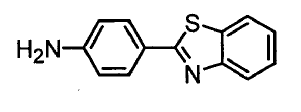

Preparation was performed according to the general procedure 2 described above and, in this case, 4-aminobenzoic acid. LC/ESI-MS: *m*/*z* = 227 [M+H].

### Synthesis of 4-benzothiazol-2-yl-2-methyl-phenylamine:

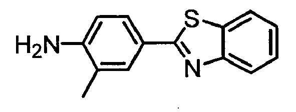

Preparation was performed according to the general procedure 2 described above and, in this case, 4-amino-3-methyl-benzoic acid. LC/ESI-MS: *m*/*z* = 241 [M+H].

### Synthesis of 3-benzothiazol-2-yl-phenylamine:

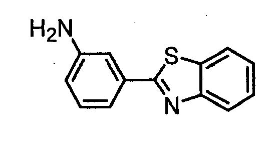

Preparation was performed according to the general procedure 2 described above and, in this case, 3-aminobenzoic acid. LC/ESI-MS: *m*/*z* = 227 [M+H].

### Synthesis of 5-benzothiazol-2-yl-2-chloro-phenylamine:

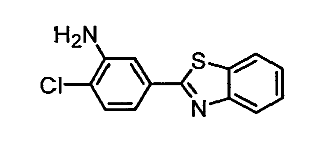

Preparation was performed according to the general procedure 2 described above and, in this case, 3-amino-4-chloro-benzoic acid. LC/ESI-MS: *m*/*z* = 261 [M+H].

### Synthesis of 4-benzothiazol-2-yl-3-methozy-phenylamine:

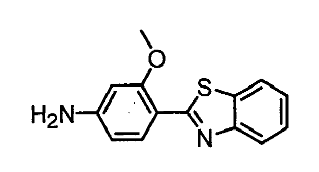

Preparation was performed according to the general procedure 2 described above and, in this case, 4-amino-2-methoxy-benzoic acid. LC/ESI-MS: *m*/*z* = 257 [M+H].

### Synthesis of (4-benzothiazol-2-yl-3-methoxy-phenyl)-methyl-amine:

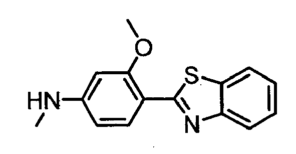

The compound was obtained as side product within the synthesis of 4-benzothiazol-2-yl-3-methoxy-phenylamine. LC/ESI-MS: *m*/*z* = 271 [M+H].

### Synthesis of 5-amino-2-benzothiazol-2-yl-phenol:

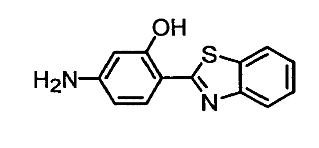

Preparation was performed according to the general procedure 2 described above and, in this case, 4-amino-2-hydroxy-benzoic acid. LC/ESI-MS: *m*/*z* = 243 [M+H].

### Synthesis of 4-amino-2-benzothiazol-2-yl-phenol:

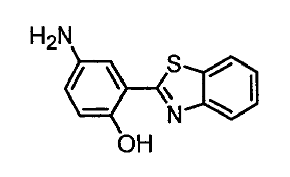

Preparation was performed according to the general procedure 2 described above and, in this case, 5-amino-2-hydroxy-benzoic acid. LC/ESI-MS: *m*/*z* = 243 [M+H].

### Synthesis of 5-benzothiazol-2-yl-2-methyl-phenylamine:

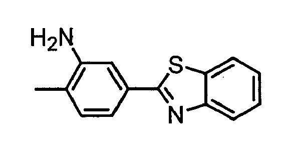

Preparation was performed according to the general procedure 2 described above and, in this case, 3-amino-4-methyl-benzoic acid. LC/ESI-MS: *m*l*z* = 241 [M+H].

### Synthesis of 4-benzothiazol-2-yl-2-trifluoromethoxy-phenylamine:

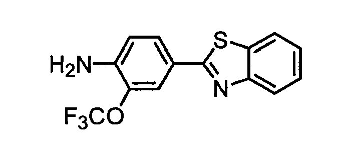

Preparation was performed according to the general procedure 2 described above and, in this case, 4-amino-3-trifluoromethoxy-benzoic acid. LC/ESI-MS: *m*/*z* = 310 [M+H].

### Synthesis of 4-benzothiazol-2-yl-3-chloro-phenylamine:

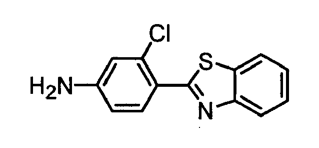

Preparation was performed according to the general procedure 2 described above and, in this case, 4-amino-2-chloro-benzoic acid. LC/ESI-MS: *m*/*z* = 261 [M+H].

### Synthesis of 4-benzothiazol-2-yl-3-fluoro-phenylamine:

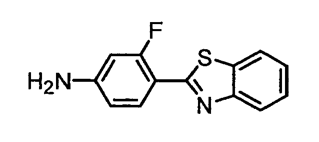

Preparation was performed according to the general procedure 2 described above and, in this case, 4-amino-2-fluoro-benzoic acid. LC/ESI-MS: *m*/*z* = 245 [M+H].

### Synthesis of 4-benzothiazol-2-yl-2-methoxy-phenylamine:

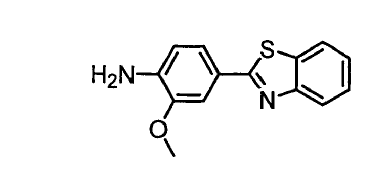

Preparation was performed according to the general procedure 2 described above and, in this case, 4-amino-3-methoxy-benzoic acid. LC/ESI-MS: *m*l*z* = 257 [M+H].

### Synthesis of 4-benzothiazol-2-yl-2-fluoro-phenylamine:

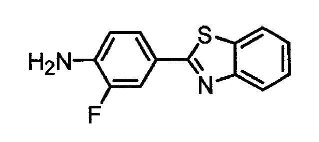

Preparation was performed according to the general procedure 2 described above and, in this case, 4-amino-3-fluoro-benzoic acid. LC/ESI-MS: *m*/*z* = 245 [M+H].

### Synthesis of 2-amino-5-benzothiazol-2-yl-phenol:

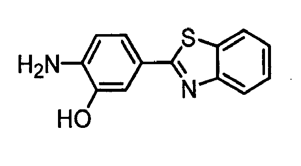

Preparation was performed according to the general procedure 2 described above and, in this case, 4-amino-3-hydroxy-benzoic acid. LC/ESI-MS: *m*/*z* = 243 [M+H].

### Synthesis of 4-(5-trifluoromethyl-benzothiazol-2-yl)-phenylamine:

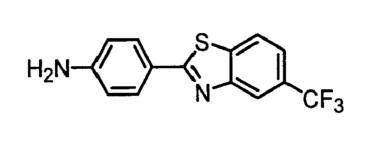

Preparation was performed according to the general procedure 2 described above and, in this case, 4-amino-benzoic acid and 2-amino-4-trifluoromethyl-benzenethiol. LC/ESI-MS: *m*/*z* = 295 [M+H].

### Synthesis of 3-benzothiazol-2-yl-4-chloro-phenylamine:

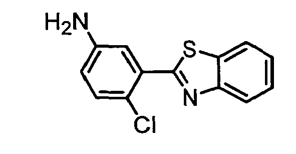

Preparation was performed according to the general procedure 2 described above and, in this case, 5-amino-2-chloro-benzoic acid. LC/ESI-MS: *m*/*z* = 261 [M+H].

### Synthesis of 2-amino-6-benzothiazol-2-yl-phenol:

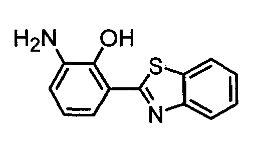

Preparation was performed according to the general procedure 2 described above and, in this case, 3-amino-2-hydroxy-benzoic acid. LC/ESI-MS: *m*/*z* = 243 [M+H].

### Synthesis of 4-(5-chloro-benzothiazol-2-yl)-phenylamine:

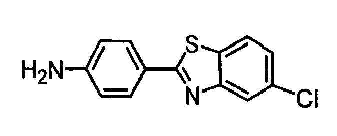

Preparation was performed according to the general procedure 2 described above and, in this case, 4-amino-benzoic acid and 2-amino-4-chloro-benzenethiol. LC/ESI-MS: *m*/*z* = 261 [M+H].

### Synthesis of 4-(6-fluoro-benzothiazol-2-yl)-phenylamine:

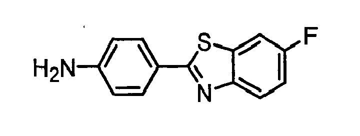

Preparation was performed according to the general procedure 3 described above and, in this case, starting from 6-fluoro-benzothiazol-2-ylamine. LC/ESI-MS: *m*/*z* = 245 [M+H].

### Synthesis of 4-(6-chloro-benzothiazol-2-yl)-phenylamine:

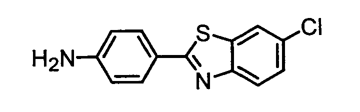

Preparation was performed according to the general procedure 3 described above and, in this case, starting from 6-chloro-benzothiazol-2-ylamine. LC/ESI-MS: *m*/*z* = 261 [M+H].

### Synthesis of 4-(6-chloro-benzothiazol-2-yl)-2-fluoro-phenylamine:

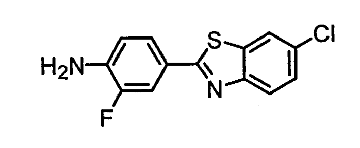

Preparation was performed according to the general procedure 3 described above and, in this case, starting from 6-chloro-benzothiazol-2-ylamine. LC/ESI-MS: *m*/*z* = 279 [M+H].

### Synthesis of 4-benzothiazol-2-yl-3-methyl-phenylamine:

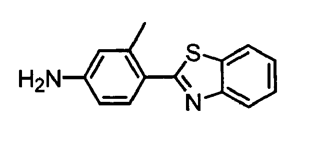

Preparation was performed according to the general procedure 2 described above and, in this case, 4-amino-2-methyl-benzoic acid. LC/ESI-MS: *m*l*z* = 241 [M+H].

### Synthesis of 4-(6-methoxy-benzothiazol-2-yl)-phenylamine:

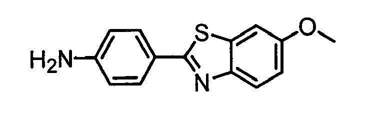

Preparation was performed according to the general procedure 3 described above and, in this case, starting from 6-methoxy-benzothiazol-2-ylamine. LC/ESI-MS: *m*/*z* = 257 [M+H].

### Synthesis of 2-fluoro-4-(6-fluoro-benzothiazol-2-yl)-phenylamine:

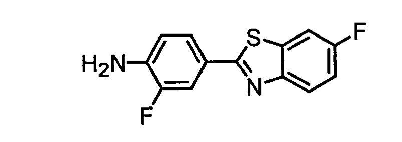

Preparation was performed according to the general procedure 3 described above and, in this case, starting from 2-amino-6-fluorobenzothiazole. LC/ESI-MS: *m*/*z* = 263 [M+H].

### Synthesis of 4-(6-bromo-benzothiazol-2-yl)-phenylamine:

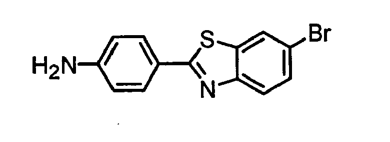

Preparation was performed according to the general procedure 3 described above and, in this case, starting from 6-bromo-benzothiazol-2-ylamine. LC/ESI-MS: *m*/*z* = 305 [M+H].

### Synthesis of 4-(6-trifluoromethoxy-benzothiazol-2-yl)-phenylamine:

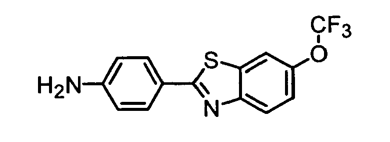

Preparation was performed according to the general procedure 3 described above and, in this case, starting from 6-trifluoromethoxy-benzothiazol-2-ylamine. LC/ESI-MS: *m*/*z* = 311 [M+H].

### Synthesis of 4-benzothiazol-2-yl-phenol:

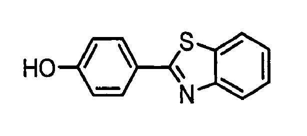

Preparation was performed according to the general procedure 2 described above and, in this case, 4-hydroxy-benzoic acid. LC/ESI-MS: *m*/*z* = 228 [M+H].

### Synthesis of 4-benzothiazol-2-yl-2-fluoro-phenol:

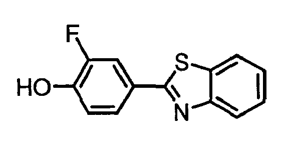

Preparation was performed according to the general procedure 2 described above and, in this case, 4-hydroxy-3-fluoro-benzoic acid. LC/ESI-MS: *m*/*z =* 246 [M+H].

### Syntheses of the Examples.

### General procedure 4:

**Reaction of 4-chloroquinazolines without alkylamino sidechains with 3- and 4-(benzothiazol-2-yl)-phenylamines** (in analogy to T. Kumagai et al., Bioorg. Med. Chem. 2001, 9, 1349-1355):
A mixture of the respective 4-chloroquinazoline (0.221 mmol) and 3- or 4-(benzothiazol-2-yl)-phenylamine (0.221 mmol), respectively, in ethylene glycol (1.5 mL) was heated to 100°C in a sealed vial for 3 h. In many cases, product precipitated upon cooling of the mixture to r.t. The resulting slurry was rinsed into a frit with ethanol, the filter cake was washed several times with the same solvent and finally with some Et₂O. If necessary, product was further washed with CHCl₃/MeOH-ultrasound and Et₂O/CHCl₃-ultrasound and separated using a centrifuge.

### General procedure 5:

### Reaction of 4-chloroquinazolines with alkylamino sidechains with 3- and 4-(benzothiazol-2-yl)-phenylamines

To a mixture of the respective 4-chloroquinazoline (0.130 mmol) and 3- or 4-(benzothiazol-2-yl)-phenylamine (0.143 mmol), respectively, in ethylene glycol (1.2 mL) was added 4 M HCl in dioxane (2.0 equiv.), and the sealed vial was heated for 3 h to 110°C, or ― in case of steric hindrance in the *ortho*-position of the amino group of 3- or 4-(benzothiazol-2-yl)-phenylamine ― to 140°C.
The reaction mixture was partitioned between satd aq. NaHCO₃/brine 1:3 (100 mL) and CHCl₃ (100 mL, then 2x50 mL). Combined org. phases were re-extracted once against 50 mL brine and dried over MgSO₄. If necessary, product was purified by preparative TLC (1 mm silica gel, CH₂Cl₂/MeOH 90:10 or prep. HPLC on reversed phase. Product was crystallized from CHCl₃/Et₂O or acetone/Et₂O.

### General procedure 6:

**Reaction of 4-chloroquinazolines with alkylamino sidechains with 3- and 4-(benzothiazol-2-yl)-phenols** (according to K. Kubo et al., J Med. Chem. 2005, 48, 1359-1366):
To a cooled solution (0 °C) of substituted 3- or 4-(benzothiazol-2-yl)-phenol (0.11 mmol) in DMSO (1 mL) was added NaH (0.11 mmol) and the mixture stirred at r.t. for 10 min. 4-Chloroquinazoline (0.11 mmol) was then added, and stirring continued at 130 °C for 12 h. Water (5 mL) was added to the reaction mixture, which was further stirred for 5 min, and the product was purified by preparative HPLC.

### General procedure 7:

**Reaction of 2- or 4-chloropyrimidines with 3- and 4-(benzothiazol-2-yl)-phenylamines** (according to T. Kumagai et al., Bioorg. Med. Chem. 2001, 9, 1349-1355):
A mixture of the respective 2- or 4-chloropyrimidine (0.221 mmol; synthesized as described above) and 3- or 4-(benzothiazol-2-yl)-phenylamine (0.221 mmol), respectively, in ethylene glycol (1.5 mL) was heated to 160°C in a sealed vial for 3 h.
If product precipitated upon cooling of the mixture to r.t., the resulting slurry was rinsed into a frit with ethanol, the filter cake was washed several times with the same solvent and finally with some Et₂O. If necessary, product was further washed with CHCl₃/acetone-ultrasound and separated using a centrifuge.
If product stayed dissolved, mixtures were partitioned between satd aq. NaHCO₃/brine (25+25 mL) and CHCl₃ (3x35 mL). Combined org. phases were dried over MgSO₄. If necessary, preparative TLC was performed (1 mm silica gel, petroleum ether/CH₂Cl₂/MeOH 30:90:20) or prep. HPLC on reversed phase. Product obtained was further washed with CHCl₃/MeOH-ultrasound and Et₂O/CHCl₃-ultrasound and separated using a centrifuge.

### General procedure 8:

**Palladium-catalyzed reaction of 2-chloro-4-(4-methyl-piperazin-1-yl)-pyrimidine with 3- and 4-(benzothiazol-2-yl)-phenylamine** (according to J. Yin et al., Org. Lett. 2004, 4, 3481-3484):
An oven-dried G4 vial was charged subsequently with Pd₂dba₃ (8.10 mg, 0.009 mmol), XANTPHOS ligand (15.3 mg, 0.027 mmol), pyrimidine (47.0 mg, 0,221 mmol), 3- or 4-(benzothiazol-2-yl)-phenylamine (60.0 mg, 0.265 mmol) and K₃PO₄ (65.7 mg, 0.309 mmol). The tube was evacuated and purged with argon and dioxane (1.0 mL) was added. The vial was sealed and heated to 100°C for 21 h.
Mixtures were filtered through a pipette stuffed with cotton and then directly mounted on a prep. TLC plate (1 mm silica gel), reaction solvent was dried away in a vigorous stream of air, and separation was achieved using petroleum ether/CH₂Cl₂/MeOH 30:90:20. Crude product was crystallized from CHCl₃/Et₂O to give products as beige powders in 20-38% yield.

### General procedure 9:

**Reaction of 6-chloropurines with 3- and 4-(benzothiazol-2-yl)-phenylamine:**
A mixture of the respective 6-chloropurine (0.221 mmol) and 3- or 4-(benzothiazol-2-yl)-phenylamine (0.243 mmol), respectively, in ethylene glycol (2.0 mL) was heated to 110°C in a sealed vial for 3 h. If reaction proceeded too sluggishly, 1.0 equiv 4 M HCl in dioxane was added and stirring continued for additional 3 h at 120 °C. Product precipitated upon cooling of the mixture to r.t. Methanol/water 1:1 (40 mL) was added to the mixtures, and the resulting precipitate was filtered off and washed with 5% aq. HCl (3 x 10 mL) and diethylether (2x10 mL). If necessary, product was further washed with CHCl₃/MeOH-ultrasound and Et₂O/CHCl₃-ultrasound and separated using a centrifuge.

### General procedure 10:

**Regioselective twofold 2,4-diamination of 2,4-dichloroquinazolines:**
A mixture of 3- or 4-(benzothiazol-2-yl)-phenylamine (1.0 equiv.), 2,4-dichloroquinazoline (1.0 equiv.) and DIEA (1.0 equiv.) in *n*-BuOH was stirred at 120°C over night.
The respective amine (2.0 equiv.) was added and the mixture was overheated to 140°C in a sealed vial for 3 h. If amine was used as a hydrochloride, additional DIEA had to be added (0.5 equiv.). For weakly nucleophilic amines like 3-amino-5-methylpyrazole, NaI (1.0 equiv.) was used as additive.

### Synthesis of (55) N⁴-(4-benzothiazol-2-yl-phenyl)-N²-(5-methyl-1H-pyrazol-3-yl)-pyridine-2,4-diamine:

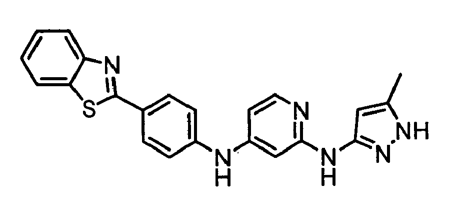

Step 1 (in analogy to S. L. Buchwald et al., J. Org. Chem. 2000, 65, 1158-1174): An oven-dried G24 vial was charged subsequently with Pd(OAc)₂ (44.0 mg, 0.196 mmol), biphenyl-2-yl-di-*tert*-butyl-phosphine ligand (114 mg, 0.392 mmol), 2-chloro-pyridin-4-ylamine (252 mg, 1.96 mmol), 2-(4-bromophenyl)-benzothiazole (683 mg, 2.35 mmol) and K₃PO₄ (583 mg, 2.74 mmol). The tube was evacuated and purged with argon and DME (4.0 mL) was added. The vial was sealed and heated to 100°C for 21 h.
The mixture was partitioned between half-saturated brine (50 mL, 1:1 H₂O/brine) and CHCl₃ (2 x 35 mL). Combined org. phases were extracted once with half-saturated brine (50 mL, 1:1 H₂O/brine) and black fluffy precipitate formed was filtered off and discarded. Combined aq. phases were extracted again with CHCl₃ (2 x 35 mL). Combined org. phases were dried over MgSO₄, mounted on silica gel and purified by chromatography on using PE/EE 20:1 to 1:2. Product fraction was further purified by prep. TLC (1 mm silica gel, PE/CH₂Cl₂/MeOH 70:70:15) and subsequent dissolving in acetone/MeOH and crushing out the product by adding H₂O. Crude product was treated with Et₂O and ultrasound, and the suspension was layered with petroleum ether to give two crystal fractions, 10% total yield of (4-benzothiazol-2-yl-phenyl)-(2-chloro-pyridin-4-yl)-amine.
Step 2: A mixture of product of step 1 (19.0 mg, 0.056 mmol) and 3-amino-5-methylpyrazole (8.20 mg, 0.084 mmol) in ethylene glycol (0.5 mL) was treated with 4 M HCl in dioxane (14 µL, 0.056 mmol) and heated to 160°C in a sealed G4 vial for 22 h. The mixture was partitioned between satd aq. NaHCO₃/brine 1:1 (50 mL) and CHCl₃ (3 x 35 mL), combined org. phases were dried over MgSO₄. Crude material was purified by prep. HPLC. Product fractions were partitioned between satd aq. NaHCO₃ (75 mL added to HPLC phase upon removal of CH₃CN) and CHCl₃ (3 x 50 mL), combined org. phases were dried over MgSO₄, and *N*⁴-(4-benzothiazol-2-yl-phenyl)-*N*²-(5-methyl-1H-pyrazol-3-yl)-pyridine-2,4-diamine was finally crystallized from CHCl₃/Et₂O.

**Synthesis of (58) *N*⁴-(4-Benzothiazol-2-yl-phenyl)-*N*²-methyl-pyridine-2,4-diamine** 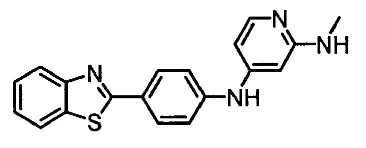 **and (86) *N*²-(4-Benzothiazol-2-yl-phenyl)-*N*²-methyl-pyridine-2,4-diamine:**

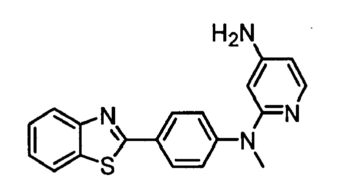

Step 1: A mixture of methylamine hydrochloride (149 mg, 2.20 mmol), 2-chloro-4-aminopyridine (257 mg, 2.00 mmol), DIEA (174 µL, 1.00 mmol) and NaI (300 mg, 2.00 mmol) in *n*BuOH (4 mL) was stirred at 120°C for 24 h.
Mixture was partitioned between NaHCO₃/brine 1:1 (50 mL) and CHCl₃ (3 x 35 mL). Combined org. phases were washed once with brine (50 mL) and dried over MgSO₄. The aq. phase still contained some product along with high quantities of DIEA, so water was removed in vacuum and the remaining salt was washed with acetone/CHCl₃ several times. Both organic fractions (first from extraction, second from salt) were purified by prep. TLC (1 mm silica gel each, CH₂Cl₂/MeOH 85:15). Product was crystallized from CHCl₃/Et₂O and yielded 227 mg (92%) of a beige powder.
Step 2 (in analogy to S. L. Buchwald et al., J. Org. Chem. 2000, 65, 1158-1174): An oven-dried G4 vial was charged subsequently with Pd₂dba₃ (36.6 mg, 0.040 mmol), biphenyl-2-yl-di-*tert*-butyl-phosphine ligand (47.8 mg, 0.160 mmol), product step 1 (98.6 mg, 0.800 mmol, 2-(4-bromophenyl)-benzothiazole (155 mg, 0.880 mmol) and sodium *tert*-pentoxide (123 mg, 1.12 mmol). The tube was evacuated and purged with argon and toluene (1.6 mL) was added. The vial was sealed and heated to 110°C for 21 h. The mixture was partitioned between half-saturated brine (50 mL, 1:1 H₂O/brine) and CHCl₃ (3 x 35 mL). Combined org. phases were dried over MgSO₄ and purified by prep HPLC. Two product fractions were collected (constitutional isomers), partitioned between satd aq. NaHCO₃ (20 mL) and CHCl₃ (3 x 30 mL) to remove any formic acid still present from HPLC and dried again over MgSO₄. Both fractions were finally purified by prep. TLC (1 mm silica gel, CH₂Cl₂/MeOH 80:20), dissolved in a few drops CH₂Cl₂ and crushed out with Et₂O to give *N*⁴-(4-Benzothiazol-2-yl-phenyl)-*N*²-methyl-pyridine-2,4-diamine and *N*²-(4-Benzothiazol-2-yl-phenyl)-*N*²-methyl-pyridine-2,4-diamine, respectively as beige solids (yields around 1% each).

### Synthesis of (56) 4-(4-benzothiazol-2-yl-phenylamino)-pyridine-2-carbogylic acid methylamide:

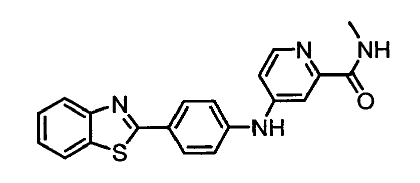

Step 1: A mixture of methylamine hydrochloride (544 mg, 8.05 mmol) and 4-chloropicolinic acid (296 mg, 2.30 mmol) in DMF (5 mL) was treated with EDC*HCl (661 mg, 3.45 mmol) and DIEA (2.0 mL, 11.5 mmol) at r.t. for 40 h. was continued over night (LCMS: ST179_21h).
Reaction mixture was partitioned between aq. satd NaHCO₃ (50 mL) and CHCl₃ (3 x 35 mL). Combined org. phases were washed with aq. satd NH₄Cl (2 x 50 mL) and dried over MgSO₄. The organic phase was purified by chromatography on silica gel using petroleum ether/ethyl acetate 2:1 1 to pure ethyl acetate. Product was finally purified by prep. HPLC. Product fraction was again partitioned between satd aq. NaHCO₃ (20 mL) and CHCl₃ (3 x 30 mL) to remove any formic acid still present from HPLC, and dried over MgSO₄. Resulting oil was dried only under reduced pressure, 25 mbar, as product is volatile at high vacuum.
Step 2: A mixture of 4-benzothiazol-2-yl-phenylamine (59.7 mg, 0.264 mmol), product step 1 (40.9 mg, 0.240 mmol) and 4.0 M HCl/dioxane (45 µL, 0.180 mmol) in DMF (0.5 mL) was heated to 160°C for 7 h.
Slurry was rinsed out into a frit (pore size 4) with EtOH, the filter cake was washed several times with the same solvent and finally with some Et₂O. Solid was treated with CHCl₃-ultrasound to remove impurities and 4-(4-benzothiazol-2-yl-phenylamino)-pyridine-2-carboxylic acid methylamide was separated using a centrifuge, resulting in 17.5 mg (20%) of a beige powder.

### Synthesis of (57) (4-benzothiazol-2-yl-phenyl)-pyridin-4-yl-amine:

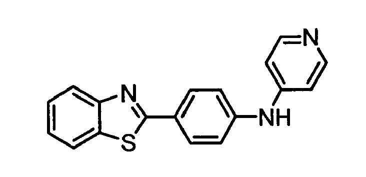

When a mixture of 4-benzothiazol-2-yl-phenylamine (79.2 mg, 0.350 mmol) and 4-chloropicolinic acid (45.0 mg, 0.350 mmol) in DMF (0.5 mL) was heated to 160°C for 3 h, decarboxylation of product formed occurred immediately, but not of starting picolinic acid.
Mixture was filtered through a pipette stuffed with cotton wool and then purified by prep. TLC (1 mm silica gel), using PE/EE 1:1. (4-Benzothiazol-2-yl-phenyl)-pyridin-4-yl-amine was finally purified by prep. HPLC to give a pale yellow solid (9.10 mg, 8%).

### General procedure 11:

**Reaction of 4-chloropyridine with substituted 3- and 4-(benzothiazol-2-yl)-phenylamines:**
A Mixture of 4-chloropyridine (40.0 mg, 0.350 mmol) and substituted 3- and 4-(benzothiazol-2-yl)-phenylamines (0.350 mmol) in DMF (2.0 mL) was heated for 3 h at 160°C.
The mixtures were dissolved in DMSO (2 mL) and separated by prep. HPLC. Product was finally purified by additional prep. TLC (1 mm silica gel, CH₂Cl₂/MeOH, 90:10).

### General procedure 12:

**Reaction of 4,7-dichloroquinoline and 4-chloro-thieno[3,2-d]pyrimidine with substituted 3- and 4-(benzothiazol-2-yl)-phenylamines:**
A mixture of 4,7-dichloroquinoline or 4-chloro-thieno[3,2-d]pyrimidine (0.242 mmol) and the respective 3- or 4-(benzothiazol-2-yl)-phenylamines (0.220 mmol) and 4.0 M HCl/dioxane (65 µL, 0.440 mmol) in ethylene glycol (1 mL) was heated to 110°C for 6 h. For reactions with 4,7-dichloroquinoline, addition of water resulted in precipitation of product, which was filtered off and washed with water, diethylether, and petroleum ether. For reactions with 4-chloro-thieno[3,2-d]pyrimidine, the mixtures were separated by prep. HPLC.

By following the methods described above, the compounds set out in the following table were prepared.

| Compound | Structure | LC/ESI-MS: [M+H] *m*/*z* = | General Procedure |
|---|---|---|---|
| 1 | 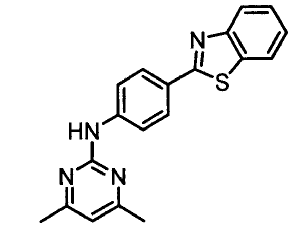 | 333 | 7 |
| 2 | 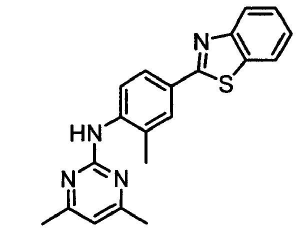 | 347 | 7 |
| 3 | 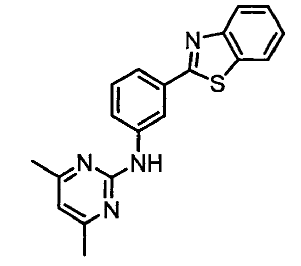 | 333 | 7 |
| 4 | 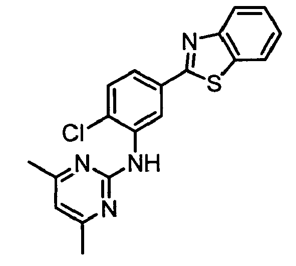 | 367 | 7 |
| 5 | 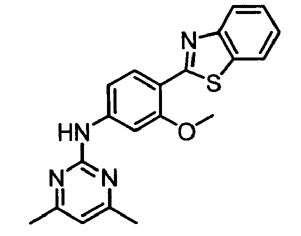 | 363 | 7 |
| 6 | 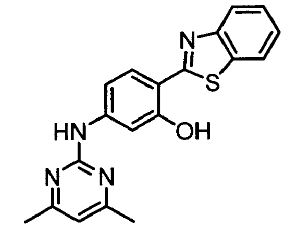 | 349 | 7 |
| 7 | 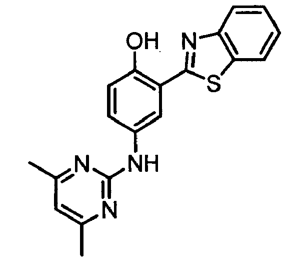 | 349 | 7 |
| 8 | 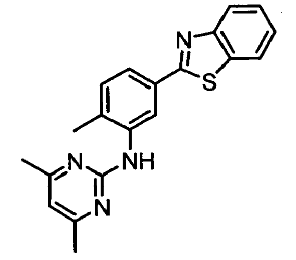 | 347 | 7 |
| 9 | 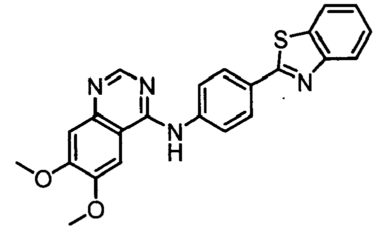 | 415 | 4 |
| 10 | 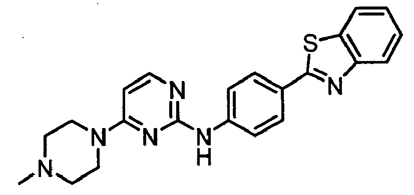 | 403 | 8 |
| 11 | 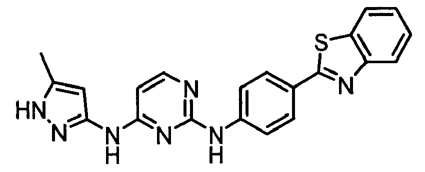 | 400 | 7 |
| 12 | 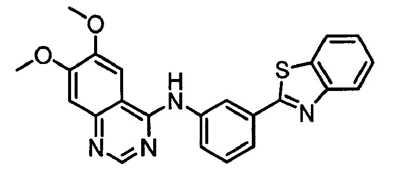 | 415 | 4 |
| 13 | 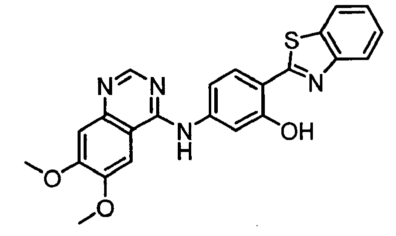 | 431 | 4 |
| 14 | 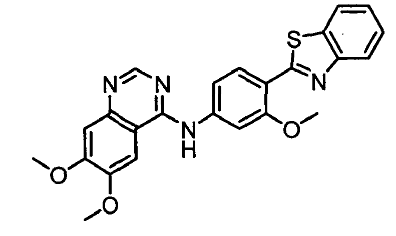 | 445 | 4 |
| 15 | 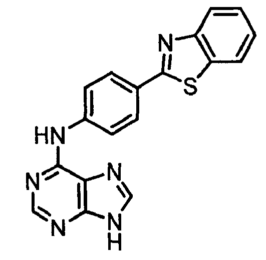 | 345 | 9 |
| 16 | 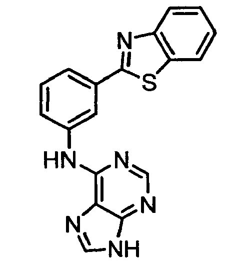 | 345 | 9 |
| 17 | 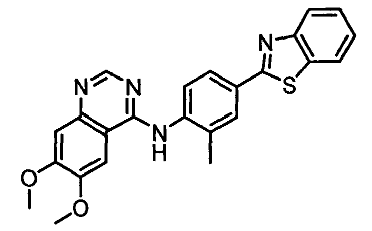 | 429 | 4 |
| 18 | 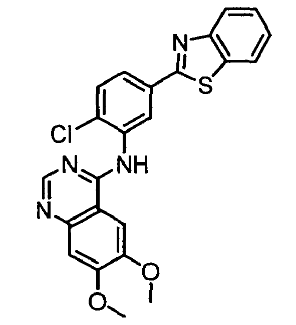 | 449 | 4 |
| 19 | 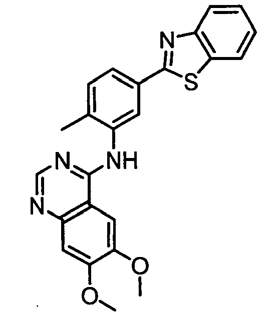 | 429 | 4 |
| 20 | 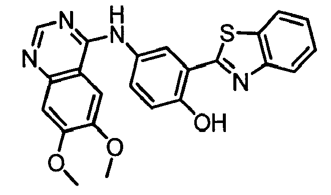 | 431 | 4 |
| 21 | 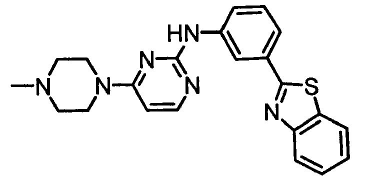 | 403 | 8 |
| 22 | 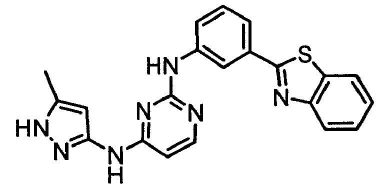 | 400 | 7 |
| 23 | 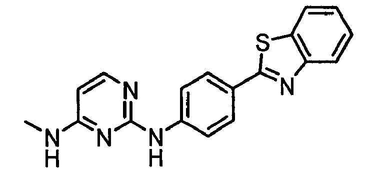 | 334 | 7 |
| 24 | 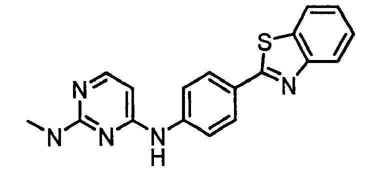 | 334 | 7 |
| 25 | 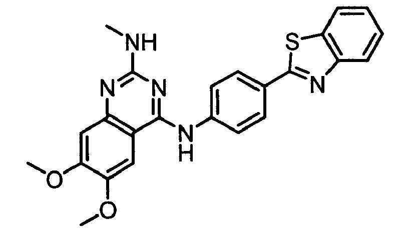 | 444 | 10 |
| 26 | 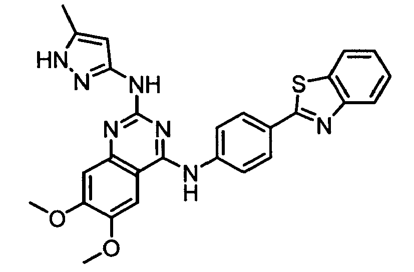 | 510 | 10 |
| 27 | 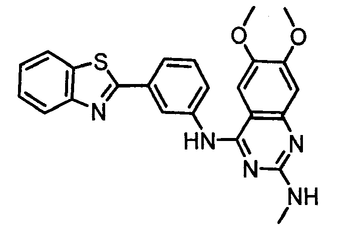 | 444 | 10 |
| 28 | 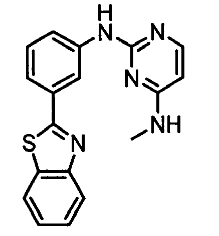 | 334 | 7 |
| 29 | 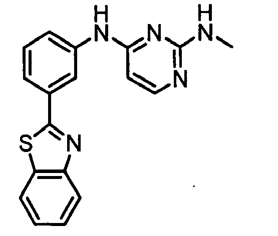 | 334 | 7 |
| 30 | 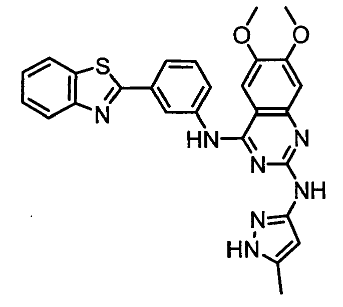 | 510 | 10 |
| 31 | 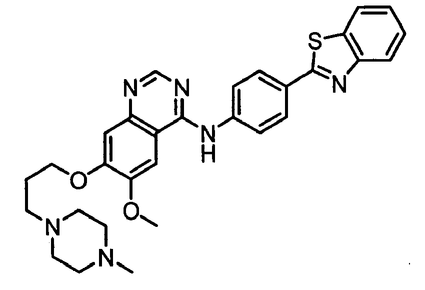 | 541 | 5 |
| 32 | 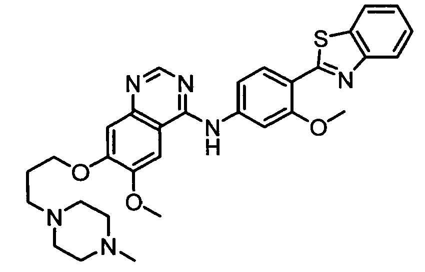 | 571 | 5 |
| 33 | 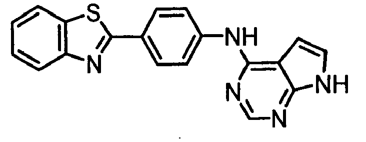 | 344 | 9 |
| 34 | 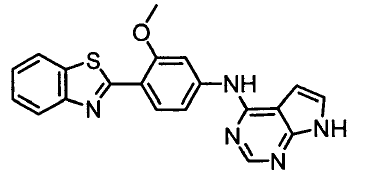 | 374 | 9 |
| 35 | 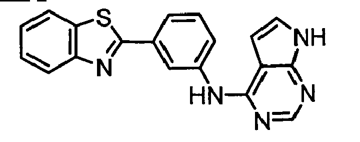 | 344 | 9 |
| 36 | 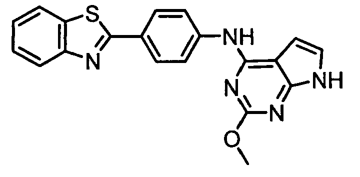 | 374 | 9 |
| 37 | 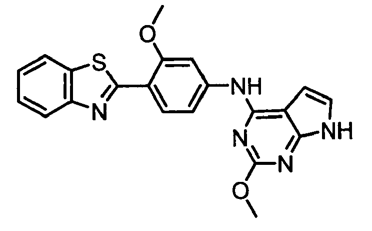 | 404 | 9 |
| 38 | 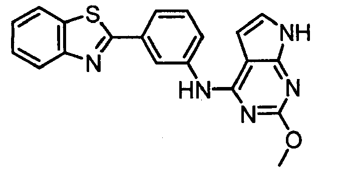 | 374 | 9 |
| 39 | 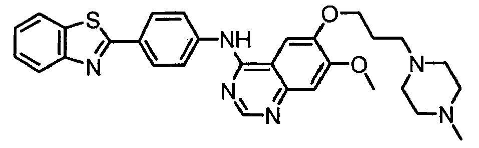 | 541 | 5 |
| 40 | 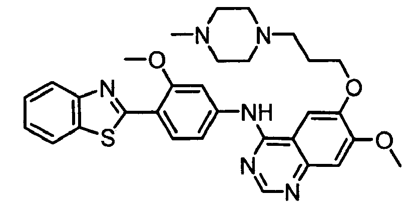 | 571 | 5 |
| 41 | 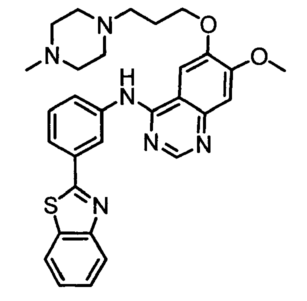 | 541 | 5 |
| 42 | 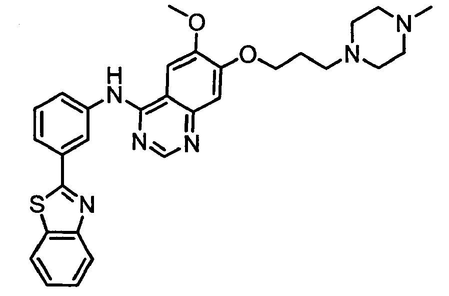 | 541 | 5 |
| 43 | 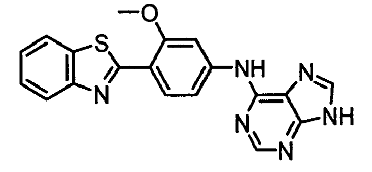 | 375 | 9 |
| 44 | 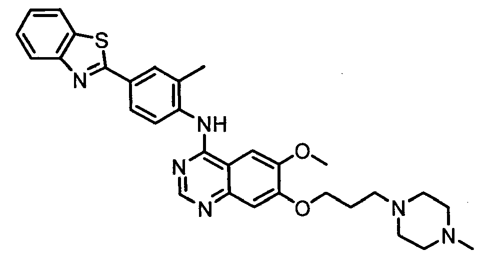 | 555 | 5 |
| 45 | 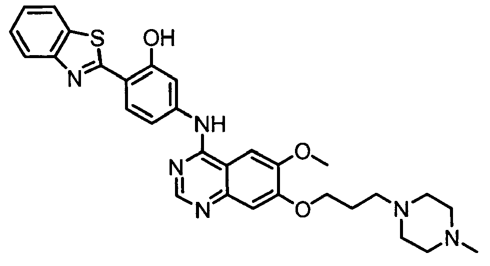 | 557 | 5 |
| 46 | 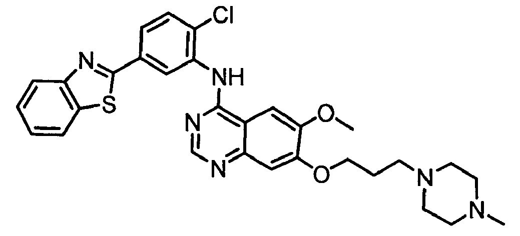 | 575 | 5 |
| 47 | 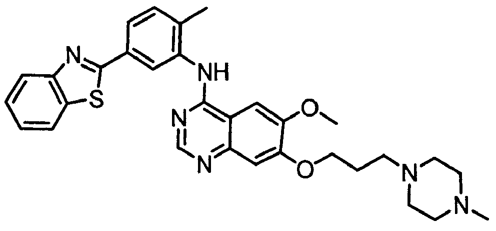 | 555 | 5 |
| 48 | 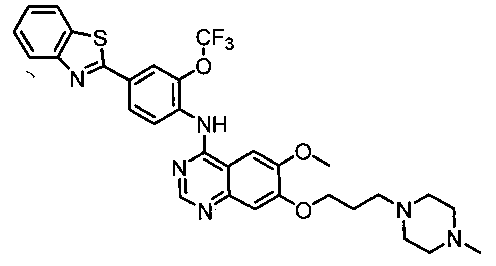 | 625 | 5 |
| 49 | 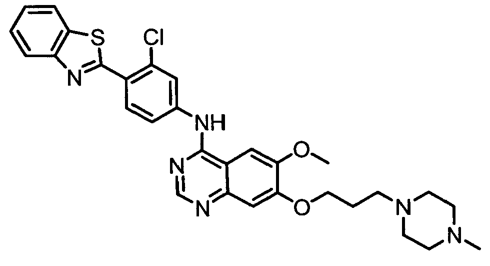 | 575 | 5 |
| 50 | 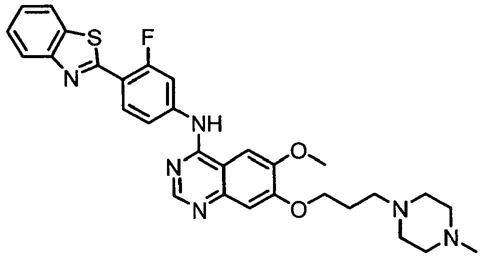 | 559 | 5 |
| 51 | 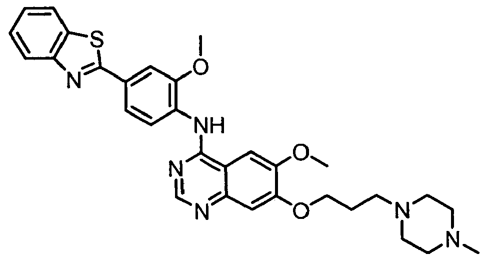 | 571 | 5 |
| 52 | 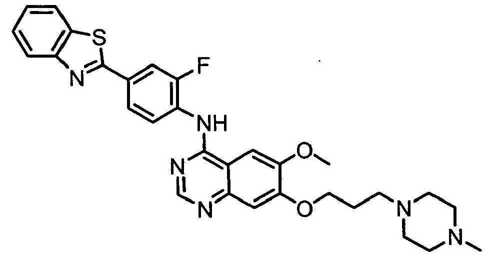 | 559 | 5 |
| 53 | 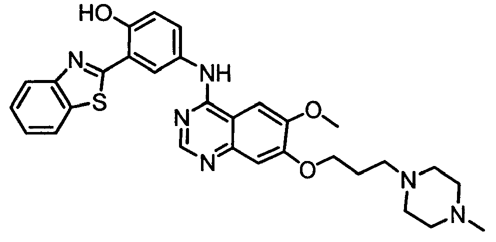 | 557 | 5 |
| 54 | 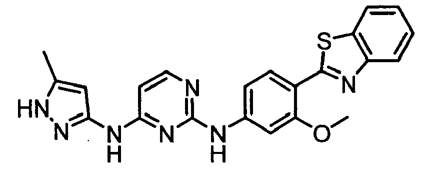 | 430 | 7 |
| 55 | 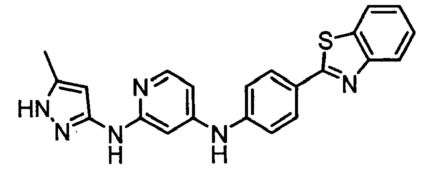 | 399 | see above |
| 56 | 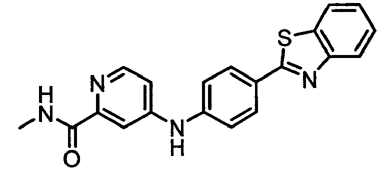 | 361 | see above |
| 57 | 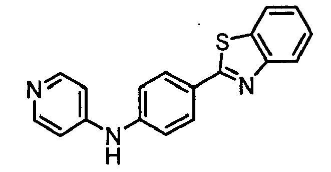 | 304 | see above |
| 58 | 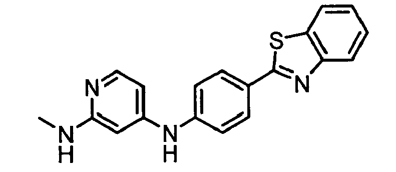 | 333 | see above |
| 59 | 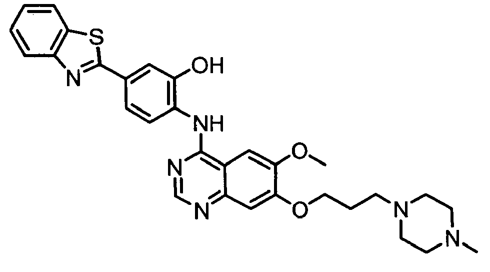 | 557 | 5 |
| 60 | 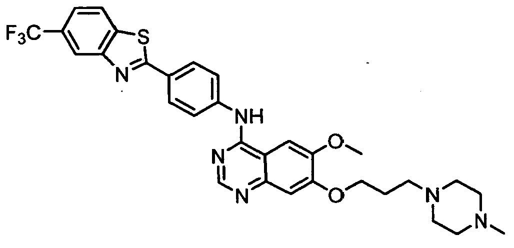 | 609 | 5 |
| 61 | 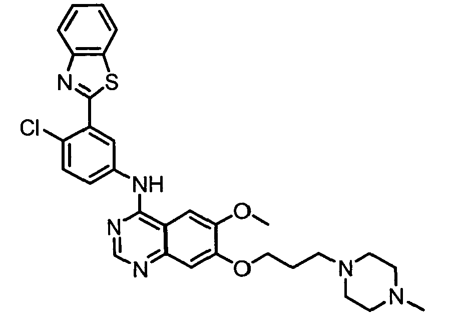 | 575 | 5 |
| 62 | 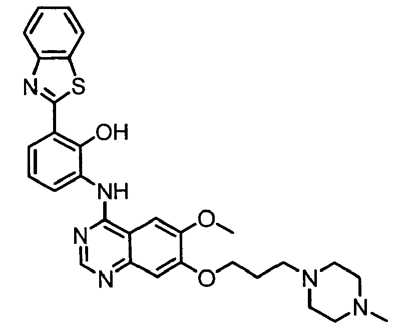 | 557 | 5 |
| 63 | 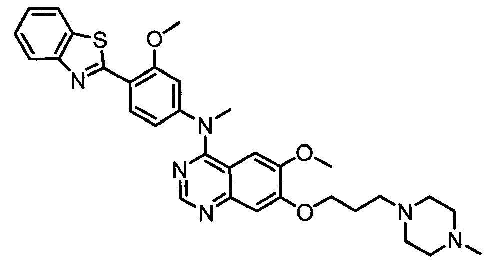 | 585 | 5 |
| 64 | 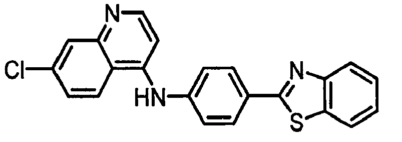 | 388 | 12 |
| 65 | 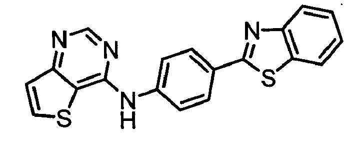 | 361 | 12 |
| 66 | 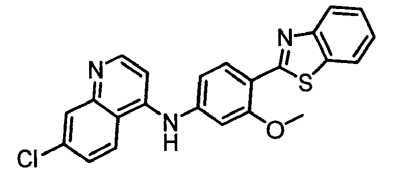 | 418 | 12 |
| 67 | 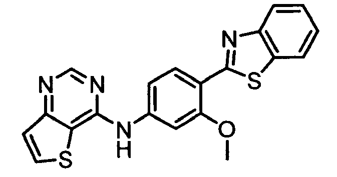 | 391 | 12 |
| 68 | 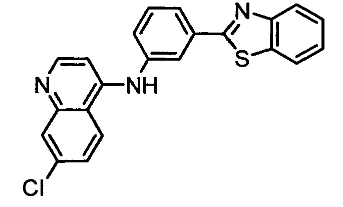 | 388 | 12 |
| 69 | 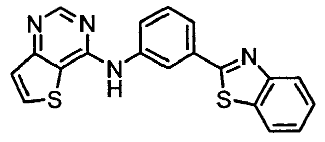 | 361 | 12 |
| 70 | 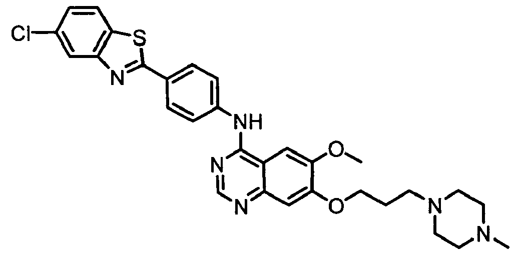 | 575 | 5 |
| 71 | 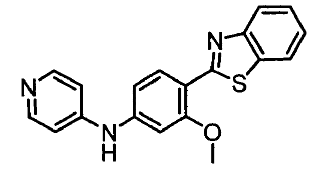 | 334 | 11 |
| 72 | 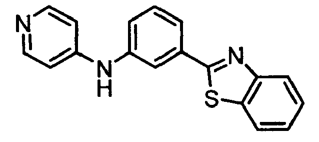 | 304 | 11 |
| 73 | 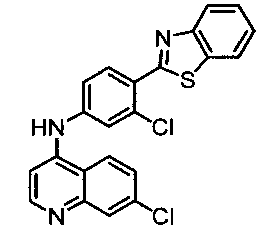 | 422 | 12 |
| 74 | 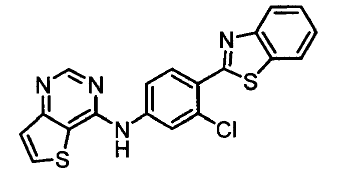 | 395 | 12 |
| 75 | 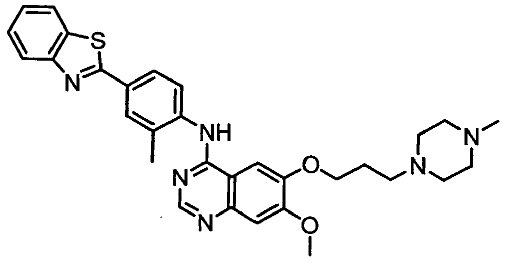 | 555 | 5 |
| 76 | 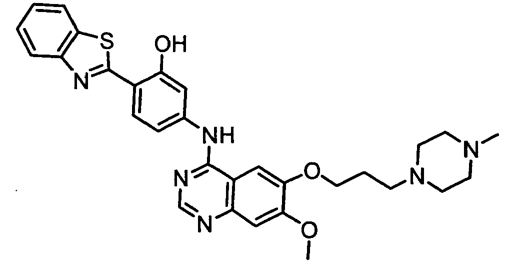 | 557 | 5 |
| 77 | 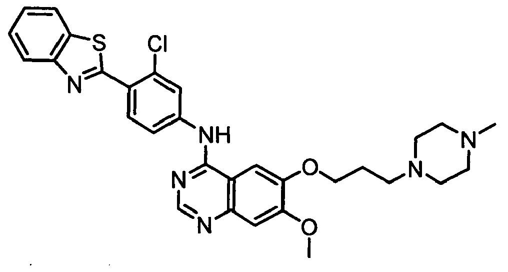 | 575 | 5 |
| 78 | 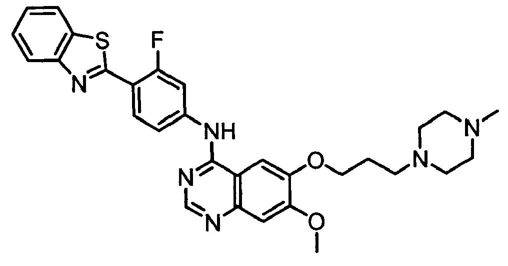 | 559 | 5 |
| 79 | 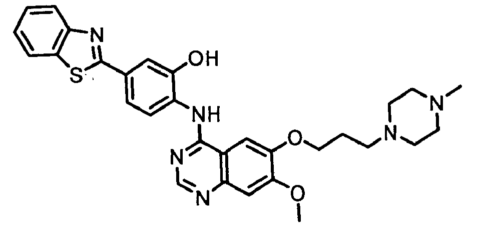 | 557 | 5 |
| 80 | 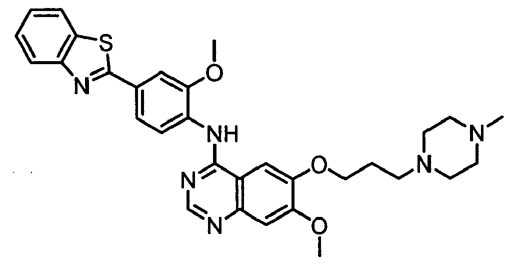 | 571 | 5 |
| 81 | 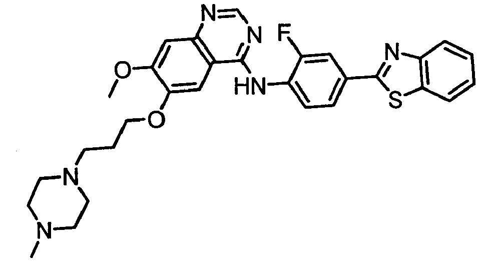 | 559 | 5 |
| 82 | 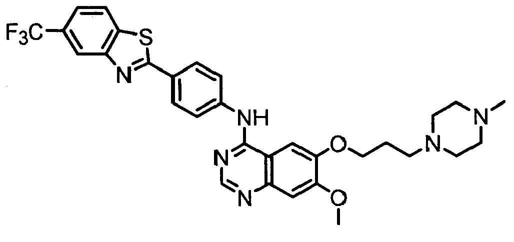 | 609 | 5 |
| 83 | 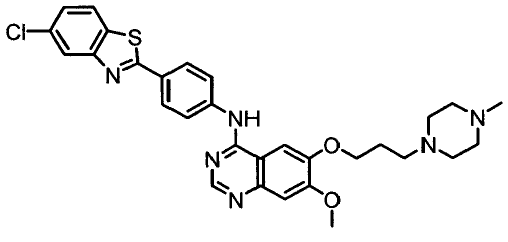 | 575 | 5 |
| 84 | 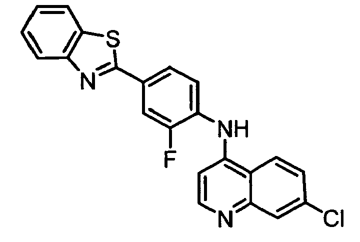 | 406 | 12 |
| 85 | 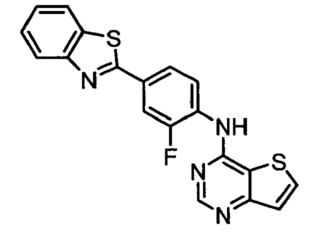 | 379 | 12 |
| 86 | 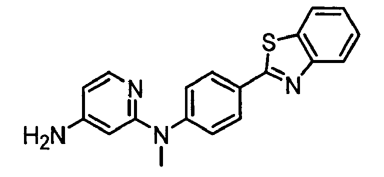 | 333 | see above |
| 87 | 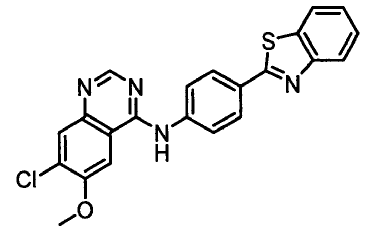 | 419 | 4 |
| 88 | 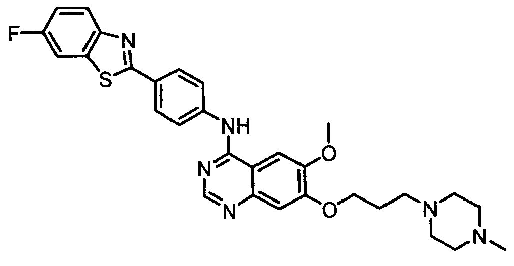 | 559 | 5 |
| 89 | 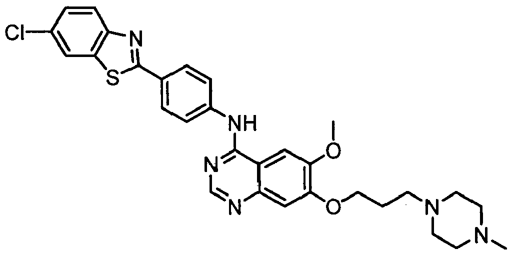 | 575 | 5 |
| 90 | 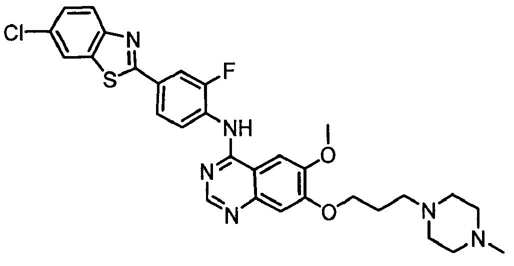 | 593 | 5 |
| 91 | 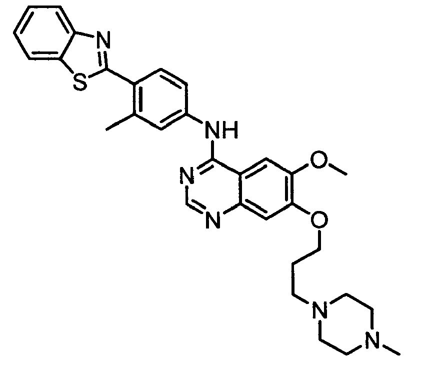 | 555 | 5 |
| 92 | 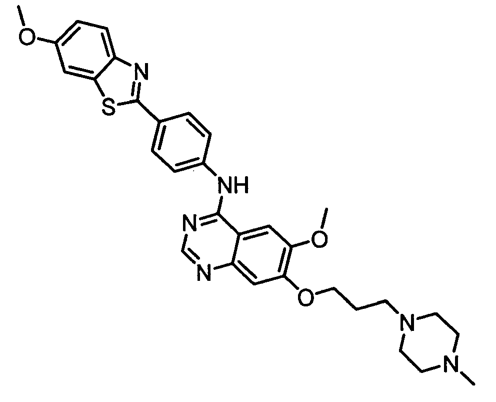 | 571 | 5 |
| 93 | 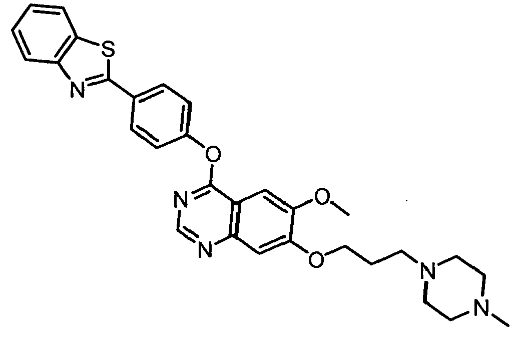 | 542 | 6 |
| 94 | 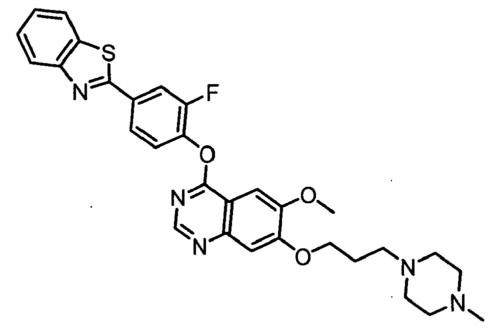 | 560 | 6 |

### Materials and methods

### In vitro Protein Kinase Assay

The effect of the 2-arylbenzothiazole derivatives was tested on recombinant, human protein kinases. All protein kinases were expressed in Sf9 insect cells as human recombinant GST-fusion proteins or as His-tagged proteins by means of the baculovirus expression system. Protein kinases were purified by affinity chromatography using either GSH-agarose or NiNTH-agarose. The purity and identity of each was checked by SDS-PAGE/silver staining and by western blot analysis with specific antibodies.
A proprietary protein kinase assay (³³PanQinase^{®} Activity Assay) was used for measuring the kinase activity. All kinase assays were performed in 96-well FlashPlates^{™} in a 50 µl reaction volume. The assay for all enzymes contained 60 mM HEPES-NaOH, pH 7.5, 3 mM MgCl₂, 3 mM MnCl₂, 3 µM Na-orthovanadate, 1.2 mM DTT, 50 µg/ml PEG₂₀₀₀₀ and 1 µM [γ-³³P]-ATP (approx. 5x10⁵ cpm per well).
The reaction cocktails were incubated at 30°C for 80 minutes. The reaction was stopped with 50 µl of 2% (v/v) H₃PO₄, plates were aspirated and washed two times with 200 µl of 0.9% (w/v) NaCl. Incorporation of ³³Pᵢ was determined with a microplate scintillation counter. All assays were performed with a BeckmanCoulter/ Sagian robotic system.

### Cellular Receptor Tyrosine Kinase Assay

The effect of 2-arylbenzothiazole derivatives was tested in cellular assays by determining the inhibition of the receptor tyrosine kinases (RTKs) of the growth factor receptors EGF-R, PDGF-R, TIE2, and VEGF-R2. To this end different cell lines expressing the respective growth factor receptor in appropriate amounts were used. 35,000 cells per well were plated in medium containing 10% fetal calf serum (FCS) in 48-well cell culture dishes. After 24 h the FCS-containing medium was exchanged against medium without FCS, and subsequently cells were starved in this medium overnight. On the next day test compounds at different concentrations in 100% DMSO were added to the cell culture medium in a 1:100 dilution step resulting in a final DMSO assay concentration of 1%. After 90 min preincubation with test compounds at 37° C, cells were stimulated at room temperature for several min with receptor-specific ligands. Receptor stimulation was followed by cell lysis using a lysis buffer complemented with standard protease and phosphatase inhibitors.
The phosphorylation status of the various RTKs was quantified in 96-well plates via a sandwich ELISA using receptor-specific capture antibodies and a generic biotinylated anti-phosphotyrosine detection antibody. Finally optical density was measured at 450 nm after addition of avidin-labelled horseradish peroxidase and Tetramethylbenzidine (TMB) as a substrate.
For each particular concentration of a test compound inhibition was calculated percentagewise relative to maximal phosphorylation in stimulated, untreated cells ("high control"). IC₅₀ values were calculated based on sigmoidal inhibitor curves covering a concentration range of 9 concentrations of each test compound in half-logarithmic steps.

### Cellular Aurora-B Kinase Assay

The effect of 2-arylbenzothiazole derivatives was tested in a cellular Aurora-B assay by measuring the effect of the test compounds on the endoreduplication of genomic DNA. Inhibition of Aurora-B results in endoreduplication of genomic DNA, which is detectable in cells as DNA-content higher then 4 n. Intercalation of fluorescent Propidium Iodine (PI) into DNA was used to quantify the DNA content by using a fluorescence activated cell sorter (FACS).
HT29 colon-carcinoma cells were seeded on day 1 of the experiment at 100,000 cells per well in 6-well cell culture dishes in 3 ml of DMEM medium containing 10% (v/v) FCS, 100 units/ml Penicillin, 100 mg/ml Streptomycin at 37°C, 10% CO₂. On day 2 test compounds at different concentrations in 100% DMSO were added to the medium in a 1:1000 dilution step resulting in a final DMSO assay concentration of 0.1%. Cells were incubated with test compounds for 3 days. On day 5 the cells were harvested by trypsinization, combined with corresponding supernatants, centrifuged, and resuspended in 80% (v/v) methanol for fixation and permeabilization at 4°C overnight. On day 6 fixed cells were centrifuged, rehydrated in PBS/1% (v/v) FCS for 1 h, and subsequently incubated with RNAse A and PI for 30 min at room temperature.
Stained cells were analyzed for DNA-content by FACS as follows. For analyses of the cell cycle distribution of the cell population, 5000 single-cell-events of the differently treated cells were aquired by FACS. DNA-intercalated PI was detected by measuring fluorescence emission using a 650 nm pass filter (FL3) upon excitation at 488 nm with an argon laser. Single cell events were plotted in a histogram according to their FL3-A signal. Signal amplification for the first peak of the FL-3 amplitude (FL3-A) was set to about 200 arbitrary units (AU). Using an untreated cell population, gates were defined for each of the different cell cycle phases. The area containing the gaussian-curve-shaped first peak at 200 AU was defined as "cells in *G1*-phase" containing the double set of chromosomes (2n). The area around the peak at 400 AU was defined as "cells in *G2*/*M*-phase" containing the quadruple set of chromosomes (4n). Events in between G1 and G2/M was defined as "cells in S-phase", those below G1 (subG1) as "apoptotic". Importantly, all events beyond the G2/M-gate were defined as "endoreduplicated cells" (EndoR). For each concentration of a test compound the percentage of EndoR-population as compared to the whole cell population was determined. For estimation of IC₅₀ values of Aurora-B inhibition the percentages of EndoR-populations were plotted versus compound concentrations.

### Results

### In vitro Protein Kinase Assay

The following examples show IC₅₀ values lower than 500 nM on at least one kinase selected from Aurora-A, Aurora-B, EGF-R, ERBB2, PDGFR, IGF1-R, VEGF-R2, VEGF-R3, EPHB4, TIE2, and SRC or display a beneficial activity profile by inhibiting at least two kinases from at least two different molecular mechanisms of tumor progression with IC₅₀ values lower than 500 nM: 31, 32, 39, 40, 44, 45, 48, 49, 50, 51, 52, 54, 59, 60, 70, 75, 76, 77, 78, 79, 81, 83, 85, 87, 88, 89, 90, 91, 92, 93, 94.

The following compounds show IC50 values lower than 10 µM in the Cellular Receptor Tyrosine Kinase Assay and / or the Cellular Aurora-B Kinase Assay: 45, 51, 93, 94.

## Claims

1. A compound of the general formula (I) and salts and physiologically functional derivatives thereof, 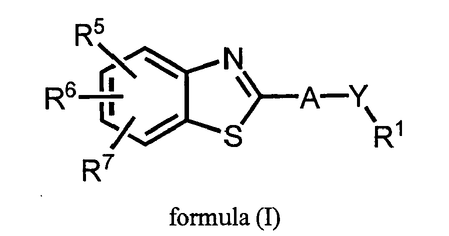 wherein
Y independently represents a divalent linkage selected from S, O, NR², SO, SO₂;
A independently represents a divalent linkage selected from a five- or six-membered aromatic carbocycle or heterocycle each of which is optionally substituted by one to four R³ or R⁴, with the proviso that A is, if Y is NR², together with Y not 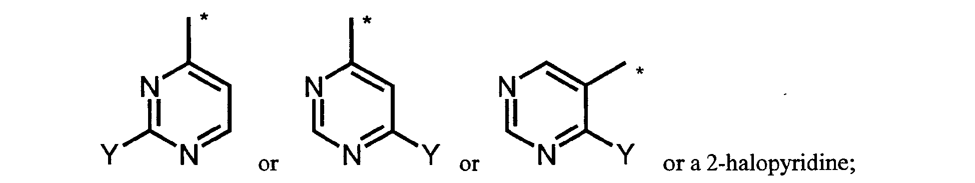
where * indicates the point of attachment to the benzothiazole;
R¹ independently represents one of the following groups:
| | | |
|---|---|---|
| 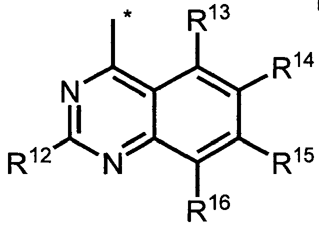 | 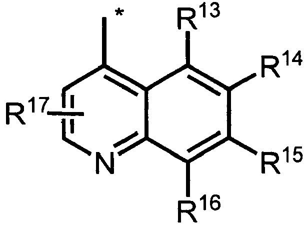 | 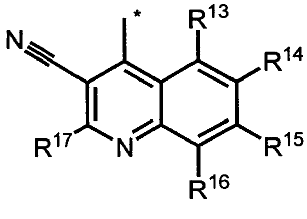 |
| 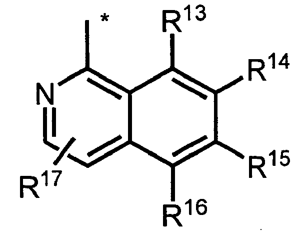 | 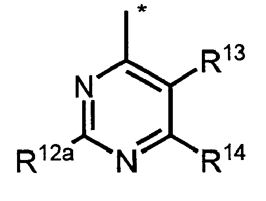 | 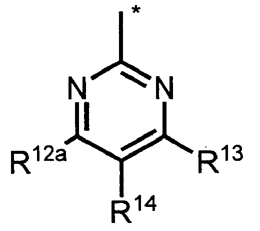 |
| 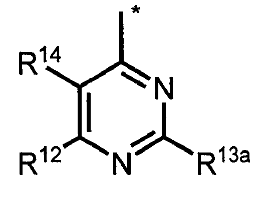 | 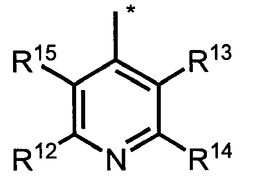 | 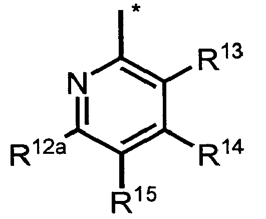 |
| 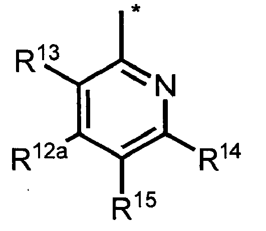 | 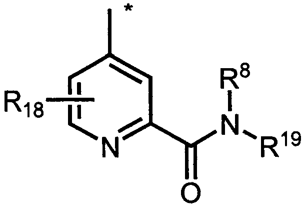 | 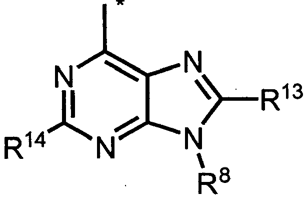 |
| 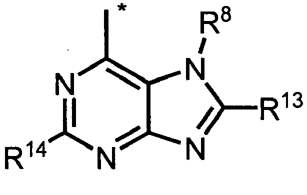 | 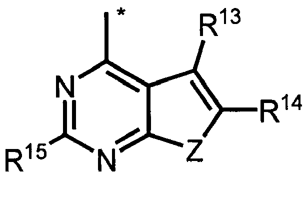 | 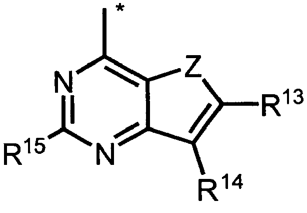 |
| 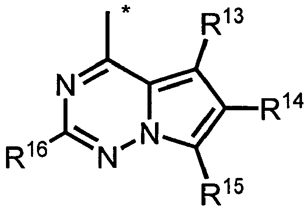 | | |
| | | |
|---|---|---|
| where * indicates the point of attachment | | |
Z independently represents O, NR⁸, S;
R² independently represents H, alkyl, cycloalkyl, -COR¹¹, -SOR¹¹, -SO₂R¹¹, -CN, hydroxyalkyl, haloalkyl, haloalkyloxy, oxyalkyl, alkoxyalkyl, aminoalkyl, alkylaminoalkyl, alkylamine, -Y^{2'}-X^{2'};
Y^{2'} independently represents C₁₋₆alkyl, C₁₋₃alkylcycloalkyl, alkyloxyalkyl, alkylaminoalkyl or alkylaryl;
X^{2'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
R³ independently represents H, -COR¹¹, -CO₂R¹¹, -SOR¹¹, -SO₂R¹¹, -SO₃R¹¹, -NO₂,-CN, -CF₃, -OCH₃, -OCF₃, alkyl, cycloalkyl, alkoxy, oxyalkyl, alkoxyalkyl, -NH₂, alkylamine, aminoalkyl, alkylaminoalkyl, -NR⁸COR¹¹, halogen, -OH, -SH, alkylthio, hydroxyalkyl, haloalkyl, haloalkyloxy, or -Y^{3'}-X^{3'};
Y^{3'} independently represents C₁₋₆alkyl, C₁₋₃alkylcycloalkyl, alkyloxyalkyl, alkylaminoalkyl or alkylaryl;
X^{3'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
R⁴ independently represents H, -COR¹¹, -CO₂R¹¹, -SOR¹¹, -SO₂R¹¹, -SO₃R¹¹, -NO₂,-CN, -CF₃, -OCH₃, -OCF₃, alkyl, cycloalkyl, alkoxy, oxyalkyl, alkoxyalkyl, -NH₂, alkylamine, aminoalkyl, alkylaminoalkyl, -NR⁸COR¹¹, halogen, -OH, -SH, alkylthio, hydroxyalkyl, haloalkyl, haloalkyloxy, -Y^{4'}-X^{4'}, substituted or unsubstituted aryl or heteroaryl;
Y^{4'} independently represents C₁₋₆alkyl, C₁₋₃alkylcycloalkyl, alkyloxyalkyl, alkylaminoalkyl or alkylaryl;
X^{4'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
R⁵ independently represents H, -COR¹¹, -CO₂R¹¹, -SOR¹¹, -SO₂R¹¹, -SO₃R¹¹, -NO₂,-CN, -CF₃, -OCH₃, -OCF₃, alkyl, cycloalkyl, alkoxy, oxyalkyl, alkoxyalkyl, -NH₂, alkylamine, aminoalkyl, alkylaminoalkyl, -NR⁸COR¹¹, halogen, -OH, -SH, alkylthio, hydroxyalkyl, haloalkyl, haloalkyloxy, -Y^{5'}-X^{5'}, substituted or unsubstituted aryl or heteroaryl;
Y^{5'} independently represents C₁₋₆alkyl, C₁₋₃alkylcycloalkyl, alkyloxyalkyl, alkylaminoalkyl or alkylaryl;
X^{5'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
R⁶ independently represents H, -COR¹¹, -CO₂R¹¹, -SOR¹¹, -SO₂R¹¹, -SO₃R¹¹, -NO₂,-CN, -CF₃, -OCH₃, -OCF₃, alkyl, cycloalkyl, alkoxy, oxyalkyl, alkoxyalkyl, -NH₂, alkylamine, aminoalkyl, alkylaminoalkyl, -NR⁸COR¹¹, halogen, -OH, -SH, alkylthio, hydroxyalkyl, haloalkyl, haloalkyloxy, -Y^{6'}-X^{6'}, substituted or unsubstituted aryl or heteroaryl;
Y^{6'} independently represents C₁₋₆alkyl, C₁₋₃alkylcycloalkyl, alkyloxyalkyl, alkylaminoalkyl or alkylaryl;
X^{6'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
R⁷ independently represents H, -COR¹¹, -CO₂R¹¹, -SOR¹¹, -SO₂R¹¹, -SO₃R¹¹, -NO₂,-CN, -CF₃, -OCH₃, -OCF₃, alkyl, cycloalkyl, alkoxy, oxyalkyl, alkoxyalkyl, -NH₂, alkylamine, aminoalkyl, alkylaminoalkyl, -NR⁸COR¹¹, halogen, -OH, -SH, alkylthio, hydroxyalkyl, haloalkyl, haloalkyloxy, -Y^{7'}-X^{7'}, substituted or unsubstituted aryl or heteroaryl;
Y^{7'} independently represents C₁₋₆alkyl, C₁₋₃alkylcycloalkyl, alkyloxyalkyl, alkylaminoalkyl or alkylaryl;
X^{7'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
R⁸ independently represents H, alkyl, cycloalkyl, -COR¹¹, -SOR¹¹, -SO₂R¹¹, hydroxyalkyl, haloalkyl, haloalkyloxy, oxyalkyl, alkoxyalkyl, aminoalkyl, alkylaminoalkyl, -Y^{8'}-X^{8'}, substituted or unsubstituted aryl or heteroaryl;
Y^{8'} independently represents C₁₋₆alkyl, C₁₋₃alkylcycloalkyl, alkyloxyalkyl, alkylaminoalkyl or alkylaryl;
X⁸' independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
R⁹ independently represents H, alkyl, cycloalkyl, hydroxyalkyl, haloalkyl, haloalkyloxy, oxyalkyl, alkoxyalkyl, aminoalkyl, alkylaminoalkyl, aryl or heteroaryl, which may be substituted;
R¹¹ independently represents H, alkyl, cycloalkyl, -NR⁸R⁹, -NR⁸NR⁸R⁹, -ONR⁸R⁹, -NR⁸OR⁹, alkylamine, arylamine, -Y^{11'}-X^{11'}, substituted or unsubstituted aryl or heteroaryl;
Y^{11'} independently represents C₁₋₆alkyl, C₁₋₃alkylcycloalkyl, alkyloxyalkyl, alkylaminoalkyl or alkylaryl;
X^{11'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
R¹² independently represents H, -NHR⁸; or one of the groups: 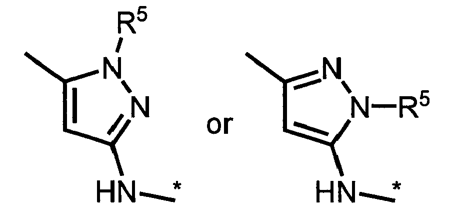 where * indicates the point of attachment;
R^{12a} independently represents one of the groups: 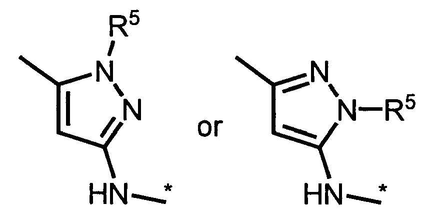 where * indicates the point of attachment;
R¹³ independently represents H, halogen, nitro, -CN, trifluoromethyl, alkyl, alkylaryl, arylalkyl, aryl, heteroaryl, -NR⁸R⁹, -Y^{13'}-X^{13'}, or -X²R¹⁸;
Y^{13'} independently represents C₁₋₆alkyl, C₁₋₃alkylcycloalkyl, alkyloxyalkyl, alkylaminoalkyl or alkylaryl;
X^{13'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl. -SO₂N(C₁₋₃alkyl)₂, -CN, -NO₂, -NH₂, -NHC₁₋₃alkyl, -N(C₁₋₃alkyl)₂, -OH, -SH;
R^{13a} independently represents H, halogen, nitro, -CN, trifluoromethyl, alkyl, alkylaryl, arylalkyl, aryl, heteroaryl, -Y^{13'}-X¹³;
R¹⁴ independently represents H, halogen, nitro, -CN, trifluoromethyl, alkyl, alkylaryl, arylalkyl, aryl, heteroaryl, -NR⁸R⁹, -Y^{14'}-X^{14'}, or -X²R¹⁸;
Y^{14'} independently represents C₁₋₆alkyl, C₁₋₃alkylcycloalkyl, alkyloxyalkyl, alkylaminoalkyl or alkylaryl;
X^{14'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂, -CN, -NO₂, -NH₂, -NHC₁₋₃alkyl, -N(C₁₋₃alkyl)₂, -OH, -SH;
R¹⁵ independently represents H, halogen, nitro, -CN, trifluoromethyl, alkyl, alkylaryl, arylalkyl, aryl, heteroaryl, -NR⁸R⁹, -Y^{15'}-X^{15'}, or -X²R¹⁸;
Y^{15'} independently represents C₁₋₆alkyl, C₁₋₃alkylcycloalkyl, alkyloxyalkyl, alkylaminoalkyl or alkylaryl;
X^{15'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂, -CN, -NO₂, -NH₂, -NHC₁₋₃alkyl, -N(C₁₋₃alkyl)₂, -OH, -SH;
R¹⁶ independently represents H, halogen, nitro, -CN, trifluoromethyl, alkyl, alkylaryl, arylalkyl, aryl, heteroaryl, -NR⁸R⁹, -Y^{16'}-X^{16'}, or -X²R¹⁸;
Y^{16'} independently represents C₁₋₆alkyl, C₁₋₃alkylcycloalkyl, alkyloxyalkyl, alkylaminoalkyl or alkylaryl;
X^{16'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂, -CN, -NO₂, -NH₂, -NHC₁₋₃alkyl, -N(C₁₋₃alkyl)₂, -OH, -SH;
R¹⁷ independently represents H, halogen, nitro, trifluoromethyl, alkyl, alkylaryl, arylalkyl, aryl, heteroaryl, -NR⁸R⁹, -Y^{17'}-X^{17'}, or -X²R¹⁸;
Y^{17'} independently represents C₁₋₆alkyl, C₁₋₃alkylcycloalkyl, alkyloxyalkyl, alkylaminoalkyl or alkylaryl;
X^{17'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
X² independently represents a direct bond, -O-, -CH₂-, -OCO-, carbonyl, -S-, -SO-, -SO₂-, -NR⁸CO-, -CONR⁸-, -SO₂NR⁸-, -NR⁸SO₂- or -NR⁸-;
R¹⁸ independently represents H, alkyl, cycloalkyl, -COR¹¹, -SOR¹¹, -SO₂R¹¹, -OCH₃,-OCF₃, hydroxyalkyl, haloalkyl, haloalkyloxy, oxyalkyl, alkoxyalkyl, aminoalkyl, alkylaminoalkyl, -Y^{18'}-X^{18'}, or one of the following groups:
| | | |
|---|---|---|
| 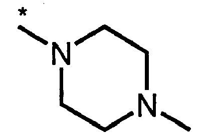 | 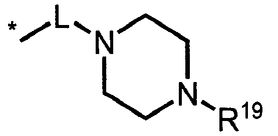 | 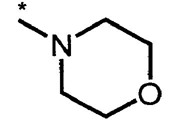 |
| 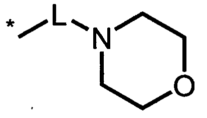 | 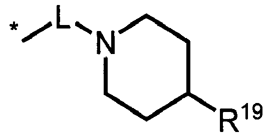 | 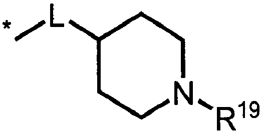 |
| 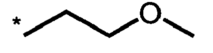 | 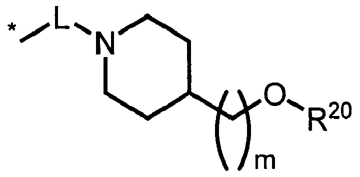 | 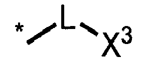 |
| 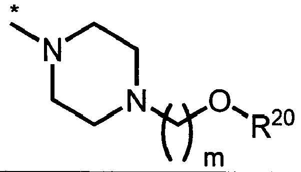 | 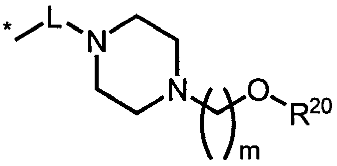 | 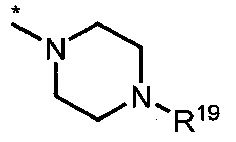 |
| 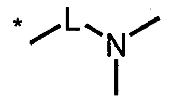 | 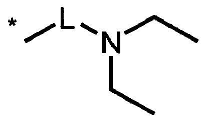 | 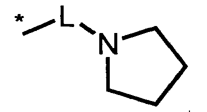 |
| 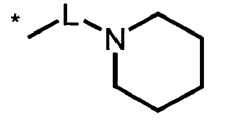 | 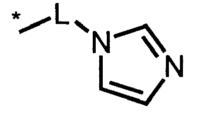 | 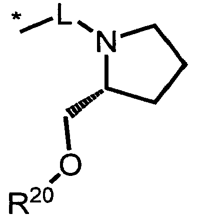 |
| 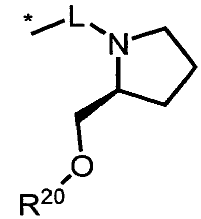 | 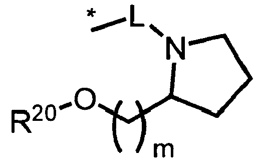 | 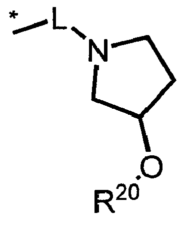 |
| 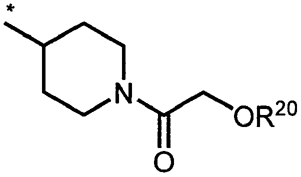 | 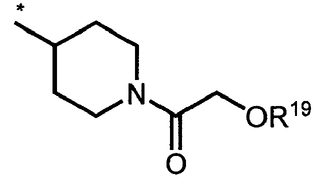 | |
| | | |
|---|---|---|
| where * indicates the point of attachment | | |
Y^{18'} independently represents C₁₋₆alkyl, C₁₋₃alkylcycloalkyl, alkyloxyalkyl, alkylaminoalkyl or alkylaryl;
X^{18'} independently represents -COOH, -COOC₁₋₃alkyl, -CONH₂, -CONHC₁₋₃alkyl, -CON(C₁₋₃alkyl)₂, -SO₃H, -SO₃C₁₋₃alkyl, -SO₂NH₂, -SO₂NHC₁₋₃alkyl, -SO₂N(C₁₋₃alkyl)₂ -CN, -NO₂, -NH₂, -NHC₁₋₃alkyl, -N(C₁₋₃alkyl)₂, -OH, -SH;
m independently represents an integer from 1-3;
L independently represents a direct bond, alkyl, cycloalkyl, alkylamine, alkyloxy, oxyalkyl, aminoalkyl, cycloalkyloxy, cycloalkylamine, ethyl, propyl, aryl or heteroaryl each of which being unsubstituted or substituted;
X³ independently represents -COOH, -COOC₁₋₆alkyl, -CONR⁸R⁹, -OH, -NR⁸R⁹, -SH, -SO₃H, -SO₂NR⁸R⁹;
R¹⁹ independently represents H, alkyl, cycloalkyl, alkylamine, alkyloxy, oxyalkyl, aminoalkyl, cycloalkyloxy, cycloalkylamine;
R²⁰ H, -PO₃H₂;
wherein an alkyl group, if not stated otherwise, denotes a linear or branched C₁-C₆-alkyl, preferably a linear or branched chain of one to five carbon atoms, a linear or branched C₂-C₆-alkenyl or a linear or branched C₂-C₆-alkynyl group, which can be substituted by one or more substituents R';
wherein R' independently represents H, -CO₂R", -CONHR", -CR"O, -SO₂NR", -NR"-CO-haloalkyl, -NO₂, -NR"-SO₂-haloalkyl, -NR"-SO₂-alkyl, -SO₂-alkyl, -NR"-CO-alkyl, -CN, alkyl, cycloalkyl, aminoalkyl, alkylamino, alkoxy, -OH, -SH, alkylthio, hydroxyalkyl, hydroxyalkylamino, halogen, haloalkyl, haloalkyloxy, aryl, arylalkyl or heteroaryl;
wherein R" independently represents H, haloalkyl, hydroxyalkyl, alkyl, cycloalkyl, aryl, heteroaryl or aminoalkyl;
wherein a cycloalkyl group denotes a non-aromatic ring system containing three to eight carbon atoms, wherein one or more of the carbon atoms in the ring can be substituted by a group E, E being O, S, SO, SO₂, N, or NR", R" being as defined above;
wherein an alkoxy group denotes an O-alkyl group, the alkyl group being as defined above;
wherein an alkylthio group denotes an S-alkyl group, the alkyl group being as defined above;
wherein an haloalkyl group denotes an alkyl group which is substituted by one to five halogen atoms, the alkyl group being as defined above;
wherein a hydroxyalkyl group denotes an HO-alkyl group, the alkyl group being as defined above;
wherein a haloalkyloxy group denotes an alkoxy group which is substituted by one to five halogen atoms, the alkyl group being as defined above;
wherein a hydroxyalkylamino group denotes an (HO-alkyl)₂-N- group or HO-alkyl-NH-group, the alkyl group being as defined above;
wherein an alkylamino group denotes an HN-alkyl or N-dialkyl group, the alkyl group being as defined above;
wherein a halogen group is chlorine, bromine, fluorine or iodine;
wherein an aryl group denotes an aromatic group having five to fifteen carbon atoms, which can be substituted by one or more substituents R', where R' is as defined above;
wherein a heteroaryl group denotes a 5- or 6-membered heterocyclic group which contains at least one heteroatom like O, N, S, wherein the heterocyclic group can be fused to another ring and can be substituted by one or more substituents R', wherein R' is as defined above.

2. The compound of claim 1, wherein A represents 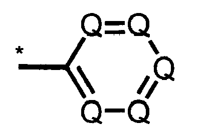 where * indicates the point of attachment to the benzothiazole
Q independently represents C, N, CH, and at least one Q represents a carbon atom.

3. The compound of claim 1 or 2, wherein R¹ is (* indicates the point of attachment).

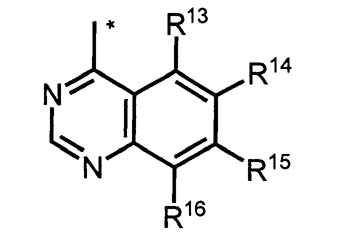

4. The compound of claim 1 or 2, wherein R¹ is (* indicates the point of attachment)

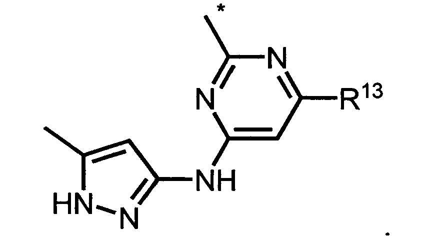

5. The compound of claim 1 or 2, wherein R¹ is (* indicates the point of attachment)

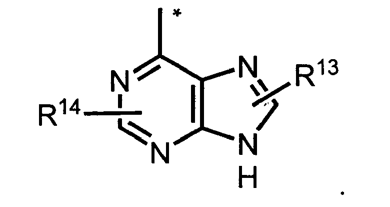

6. The compound of any one of claims 1 to 5, wherein Y is NR².

7. The compound of any one of claims 1 to 5, wherein Y is O.

8. The compound of any one of claims 2 to 7, wherein Q is C or CH and R³ is F.

9. The compound of any one of claims 2 to 7, wherein Q is C or CH and R³ is OMe.

10. The compound of any one of claims 2 to 7, wherein Q is C or CH and R³ is Cl.

11. The compound of any one of claims 2 to 10, wherein Y is attached at the 4-position of the cycle A.

12. The compound of any one of claims 2 to 10, wherein Y is attached at the 3-position of the cycle A.

13. A compound according to any one of claims 1 to 12 for the use as a medicament.

14. A compound according to any one of claims 1 to 12 for the use in therapy.

15. The use of a compound according to any one of claims 1 to 12, optionally together with appropriate adjuvants and additives, for the preparation of a medicament for the treatment or prevention of a disease selected from cell proliferation disorders, cardiovascular disorders, immunological diseases, inflammatory diseases, neuroimmunological diseases, neurodegenerative disorders, autoimmune diseases in a mammal, including a human.

16. The use of a compound according to any one of claims 1 to 12, optionally together with appropriate adjuvants and additives, for the preparation of a medicament for treating, relieving, and/or preventing cancer.

17. The use of a compound according to any one of claims 1 to 12, optionally together with appropriate adjuvants and additives, for the preparation of a medicament for treating, relieving, and/or preventing solid tumors selected from breast, bladder, colorectal, lung, prostate, pancreatic and renal cancer, or leukemias or lymphomas.

18. The use of a compound according to any one of claims 1 to 12 for the preparation of a pharmaceutical composition for the treatment of diseases which are cured or relieved by the inhibition of one or several kinases.

19. The use of a compound according to any one of claims 1 to 12 for the preparation of a pharmaceutical composition for the treatment of diseases which are cured or relieved by the inhibition of one or several kinases selected from Aurora-A, Aurora-B, EGF-R, ERBB2, PDGFR, IGF1-R, VEGF-R2, VEGF-R3, EPHB4, TIE2, and SRC.
